(19) Europäisches Patentamt
European Patent Office
Office européen des brevets

(11) Veröffentlichungsnummer: **0 315 050 B1**

# (12) EUROPÄISCHE PATENTSCHRIFT

(45) Veröffentlichungstag der Patentschrift: **01.09.93**

(51) Int. Cl.[5]: **C07C 43/18**, C09K 19/30, C09K 19/42

(21) Anmeldenummer: **88117898.2**

(22) Anmeldetag: **27.10.88**

## (54) Alkenylbicyclohexane.

(30) Priorität: **06.11.87 CH 4354/87**
**24.08.88 CH 3137/88**

(43) Veröffentlichungstag der Anmeldung:
**10.05.89 Patentblatt 89/19**

(45) Bekanntmachung des Hinweises auf die Patenterteilung:
**01.09.93 Patentblatt 93/35**

(84) Benannte Vertragsstaaten:
**CH DE FR GB IT LI NL**

(56) Entgegenhaltungen:
**WO-A-87/04426**

**CHEMICAL ABSTRACTS, Band 101, Nr. 13, 24. September 1984, Seite 610, Zusammenfassung Nr. 110419e, Columbus, Ohio, US; & JP-A-59 25 338**

**CHEMICAL ABSTRACTS, Band 105, Nr. 26, 29. Dezember 1986, Seite 615, Zusammenfassung Nr. 235974w, Columbus, Ohio, US; & JP-A-61 27 928**

(73) Patentinhaber: **F. HOFFMANN-LA ROCHE AG**
**Postfach 3255**
**CH-4002 Basel(CH)**

(72) Erfinder: **Buchecker, Richard, Dr.**
**Felsenstrasse 10**
**CH-8008 Zürich(CH)**
Erfinder: **Schadt, Martin. Dr.**
**Liestalerstrasse 77**
**CH-4411 Seltisberg(CH)**

(74) Vertreter: **Cottong, Norbert A. et al**
**Grenzacherstrasse 124 Postfach 3255**
**CH-4002 Basel (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Die vorliegende Erfindung betrifft neue Alkenylbicyclohexane, flüssigkristalline Gemische, die diese Verbindungen enthalten, sowie die Verwendung für elektro-optische Zwecke.

Flüssige Kristalle werden vor allem als Dielektrika in Anzeigevorrichtungen verwendet, da die optischen Eigenschaften solcher Substanzen durch eine angelegte Spannung beeinflusst werden können. Elektrooptische Vorrichtungen auf der Basis von Flüssigkristallen sind dem Fachmann bestens bekannt und können auf verschiedenen Effekten beruhen. Derartige Vorrichtungen sind beispielsweise Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Felder: und elektromagnetischer Strahlung besitzen. Ferner sollten die Flüssigkristallmaterialien niedere Viskosität aufweisen und in den Zellen kurze Ansprechzeiten, tiefe Schwellungsspannungen und einen hohen Kontrast ergeben. Weiterhin sollten sie bei üblichen Betriebstemperturen, d.h. in einem möglichst breiten Bereich unterhalb und oberhalb Raumtemperatur eine geeignete Mesophase besitzen, beispielsweise für die oben genannten Zellen eine nematische oder cholesterische Mesophase. Da Flüssigkristalle in der Regel als Mischungen mehrerer Komponenten zur Anwendung gelangen, vgl. WO 87/04426 oder JP-A-6 127 928, ist es wichtig, dass die Komponenten untereinander gut mischbar sind. Weitere Eigenschaften, wie die elektrische Leitfähigkeit, die dielektrische Anisotropie und die optische Anisotropie, müssen je nach Zellentyp und Anwendungsgebiet unterschiedlichen Anforderungen genügen. Beispielweise sollten Materialien für Zellen mit verdrillt nematischer Struktur eine positive dielektrische Anisotropie und eine möglichst geringe elektrische Leitfähigkeit aufweisen.

In letzter Zeit besteht ein vermehrtes Interesse an Materialien mit niedriger optischer Anisotropie, insbesondere für aktiv adressierte Flüssigkristallanzeigen, wie MIM-Anwendungen (Metall-Isolator-Metall) oder TFT-Anwendungen ("thin film transistor" = Dünnfiltransistor) in Fernsehgeräten. Die bekannten flüssigkristallinen Verbindungen mit niedriger optischer Anisotropie besitzen jedoch meist hochgeordnete smektische Phasen oder führen zu einer unerwünschten Erhöhung der Schwellenspannung, der Viskosität und/oder der Ansprechzeiten.

Gegenstand der Erfindung sind die Verbindungen der allgemeinen Formel

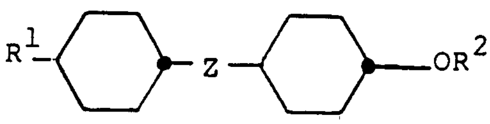

I

worin Z eine einfache Kovalenzbindung oder $-CH_2CH_2-$darstellt, $R^1$ 1E-Alkenyl mit 2-10 Kohlenstoffatomen oder 3E-Alkenyl mit 4-10 Kohlenstoffatomen bezeichnet und $R^2$ Alkyl mit 1-10 Kohlenstoffatomen, 2E-Alkenyl mit 3-10 Kohlenstoffatomen oder 3-Alkenyl mit 4-10 Kohlenstoffatomen bedeutet.

Die erfindungsgemässen Verbindungen sind Flüssigkristalle mit sehr niedriger optischer Anisotropie und vergleichsweise hohen Klärpunkten. Im Gegensatz zu vorbekannten Bicyclohexanen besitzen sie eine relativ breite nematische Mesophase oder im Falle der optisch aktiven Verbindungen eine cholesterische Mesophase. Hochgeordnete smektische Phasen werden unterdrückt. Ferner besitzen die erfindungsgemässen Verbindungen vergleichsweise niedrige Viskositäten und ermöglichen rasche Schaltzeiten. Sie sind gut löslich in anderen Flüssigkristallmaterialien und eignen sich insbesondere als Komponenten nematischer und cholesterischer Gemische.

Formel I umfasst die Verbindungen der allgemeinen Formeln

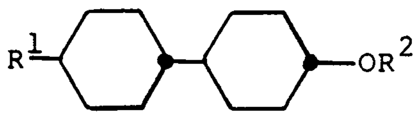

Ia

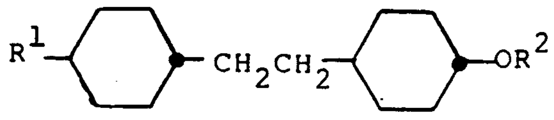

Ib

worin $R^1$ und $R^2$ die obigen Bedeutungen haben.

$R^1$ umfasst die geradkettigen Reste Vinyl, 1E-Propenyl, 1E-Butenyl, 1E-Pentenyl, 1E-Hexenyl, 1E-Heptenyl, 1E-Octenyl, 1E-Nonenyl, 1E-Decenyl, 3-Butenyl, 3E-Pentenyl, 3E-Hexenyl, 3E-Heptenyl, 3E-Octenyl, 3E-Nonenyl und 3E-Decenyl sowie verzweigt-kettige, gegebenenfalls optisch aktive Reste wie 5-Methyl-3E-heptenyl und dergleichen. Die geradkettigen Reste sind im allgemeinen bevorzugt. Ferner sind im allgemeinen 1E-Alkenylreste mit 2-7 Kohlenstoffatomen und 3E-Alkenylreste mit 4-7 Kohlenstoffatomen bevorzugt.

Der Ausdruck "Alkyl" für $R^2$ umfasst die geradkettigen Reste Methyl, Aethyl, Propyl, Butyl, Pentyl, Hexyl, Heptyl, Octyl, Nonyl und Decyl sowie verzweigt-kettige, gegebenenfalls optisch aktive Reste wie sec-Butyl, 2-Methylbutyl, 3-Methylpentyl, 4-Methylhexyl und dergleichen.

Der Ausdruck "2E-Alkenyl" für $R^2$ umfasst die geradkettigen Reste Alkyl, 2E-Butenyl, 2E-Pentenyl, 2E-Hexenyl, 2E-Heptenyl, 2E-Octenyl, 2E-Nonenyl und 2E-Decenyl sowie verzweigt-kettige, gegebenenfalls optisch aktive Reste wie 4-Methyl-2E-hexenyl und dergleichen.

Der Ausdruck "3-Alkenyl" für $R^2$ umfasst die geradkettigen Reste 3-Butenyl, 3-Pentenyl, 3-Hexenyl, 3-Heptenyl, 3-Octenyl, 3-Nonenyl und 3-Decenyl sowie verzweigt-kettige Reste wie 5-Methyl-3-heptenyl und dergleichen. Im Falle von Resten, die in E- oder Z-Form vorliegen können, ist im allgemeinen die Z-Form bevorzugt, d.h. bevorzugte Reste sind 3-Butenyl, 3Z-Pentenyl, 3Z-Hexenyl etc.

Rest $R^2$ besitzt vorzugsweise bis zu 6 Kohlenstoffatome. In obigen Formeln I, Ia und Ib bedeutet $R^2$ somit vorzugsweise Alkyl mit 1-6 Kohlenstoffatomen, 2E-Alkenyl mit 3-6 Kohlenstoffatomen oder 3-Alkenyl mit 4-6 Kohlenstoffatomen.

Von den Verbindungen der Formeln I, Ia und Ib sind im allgemeinen diejenigen bevorzugt, worin $R^1$ und $R^2$ geradkettige Reste bedeuten.

Im allgemeinen sind diejenigen Verbindungen der Formeln I, Ia und Ib bevorzugt, worin $R^1$ einen 3E-Alkenylrest bezeichnet. Die Verbindungen der Formeln I, Ia und Ib, worin $R^1$ eine 1E-Alkenylrest bezeichnet, sind jedoch u.a. dann bevorzugt, wenn kurze Seitenketten erwünscht sind.

Die optisch aktiven Verbindungen der Formeln I, Ia und Ib eignen sich als chirale Dotierstoffe. Im allgemeinen sind diejenigen bevorzugt, worin $R^1$ einen geradkettigen Rest und $R^2$ einen verzweigt-kettigen, optisch aktiven Rest bedeutet.

Die Verbindungen der Formel I können in an sich bekannter Weise hergestellt werden, beispielsweise durch Verätherung des entsprechenden Cyclohexanol-Derivates mit einer Verbindung der Formel $R^2X$, worin $R^2$ die obige Bedeutung hat und X Chlor, Brom oder Jod bezeichnet, oder durch Bildung der Gruppe $R^1$ in einer Wittig-Reaktion mit Alkyl-triphenylphosphoniumchlorid oder -bromid ausgehend vom entsprechenden Cyclohexancarboxaldehyd bzw. 3-Cyclohexylpropionaldehyd. Die Herstellung kann unter den für diese Methode üblichen Bedingungen erfolgen und ist in den Synthesebeispielen näher veranschaulicht.

Die Verbindungen der Formel I können in Form von Gemischen untereinander und/oder mit anderen Flüssigkristallkomponenten verwendet werden. wie z.B. mit Substanzen aus den Klassen der Schiffschen Basen, Azobenzole, Azoxybenzole, Phenylbenzoate, Cyclohexancarbonsäurephenylester, Cyclohexancarbonsäurecyclohexylester, Biphenyle, Phenylcyclohexane, Cyclohexylcyclohexane, Phenylpyrimidine, Cyclohexylpyrimidine, Phenyldioxane, 2-Cyclohexyl-1-phenyläthane, Terphenyle, Cyclohexylbiphenyle, Cyclohexylphenylpyrimidine und dergleichen. Derartige Substanzen sind dem Fachmann bekannt und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. Eine zweite und gegebenenfalls weitere Komponenten können weitere Verbindungen der Formel I und/oder andere Flüssigkristallkomponenten sein.

Die Verbindungen der Formel I eignen sich insbesondere für nematische oder, sofern mindestens eine Komponente des Gemisches optisch aktiv ist, auch für cholesterische Gemische. Ein bevorzugter Anwendungsbereich betrifft die Verwendung als Dielektrikum in Flüssigkristallanzeigevorrichtungen mit verdrillt nematischer Flüssigkristallstruktur, wie TN-Zellen, STN-Zellen, SBE-Zellen und OMI-Zellen. Bevorzugte Gemische sind daher diejenigen, welche eine oder mehrere Verbindungen der Formel I und eine oder mehrere Verbindungen mit positiver dielektrischer Anisotropie enthalten.

Besonders bevorzugt ist die Verwendung als Dielektrikum in Flüssigkristallanzeigevorrichtungen mit hochverdrillter nematischer Flüssigkristallschicht, wobei als hochverdrillte Flüssigkristallschichten solche zu verstehen sind, die um mehr als 90° verdrillt sind. Für diesen Anwendungsbereich ist das flüssigkristalline Gemisch zweckmässigerweise dadurch gekennzeichnet, dass es eine oder mehrere Verbindungen der Formel I und eine oder mehrere Verbindungen mit positiver dielektrischer Anisotropie enthält und mindestens eine Komponente des Gemisches optisch aktiv ist. Die optisch aktive Komponente bzw. Komponenten können Verbindungen der Formel I, Verbindungen mit positiver dielektrischer Anisotropie und/oder andere, übliche Dotierstoffe sein, wie optisch aktive Biphenyle, Ester und dergleichen. Das optisch aktive Material wird zweckmässigerweise in einer Menge verwendet, dass das Verhältnis zwischen der Schichtdicke (in der Zelle) und der natürlichen Ganghöhe des Gesamtgemisches etwa 0,2 bis 1,3 beträgt, d.h. dass bei den heute üblichen Schichtdicken von etwa 5-10 μm die natürliche Ganghöhe etwa 4-50 μm beträgt.

Aufgrund der guten Löslichkeit der Verbindungen der Formel I in anderen Flüssigkristallmaterialien und aufgrund ihrer guten Mischbarkeit untereinander kann der Anteil der Verbindungen der Formel I in den erfindungsgemässen Gemischen relativ hoch sein und beispielsweise etwa 1-70 Gew.-% betragen. Im allgemeinen ist ein Anteil von etwa 3-40 Gew.-%, insbesondere etwa 5-30 Gew.-% an Verbindungen der Formel I bevorzugt.

Vorzugsweise enthalten die erfindungsgemässen Gemische neben einer oder mehreren Verbindungen der Formel I eine oder mehrere Verbindungen aus der Gruppe der Verbindungen der allgemeinen Formeln

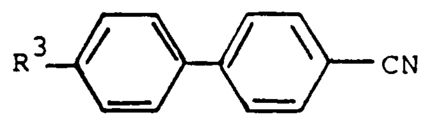

II

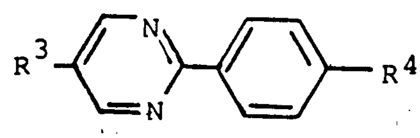

III

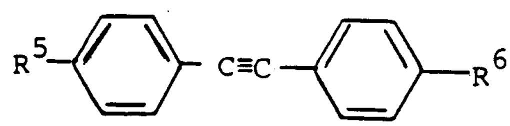

IV

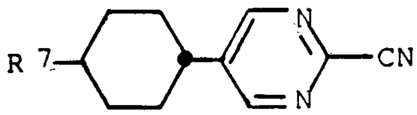

V

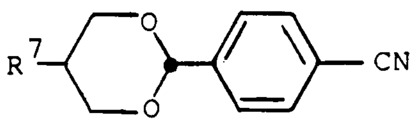

VI

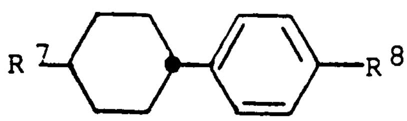

VII

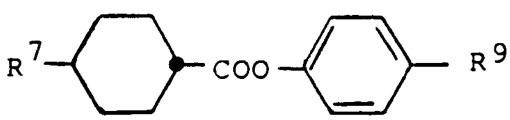

VIII

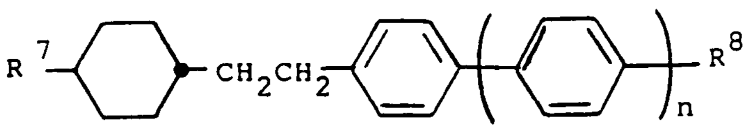

IX

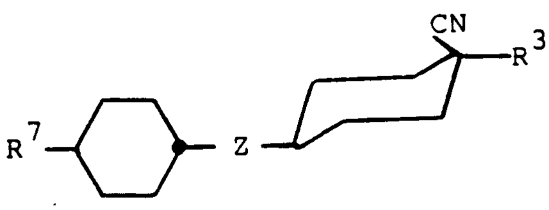

X

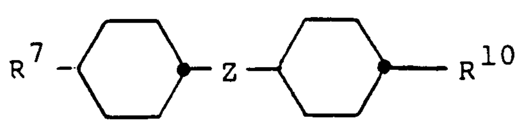

XI

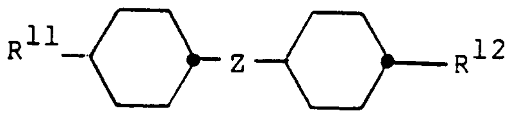

XII

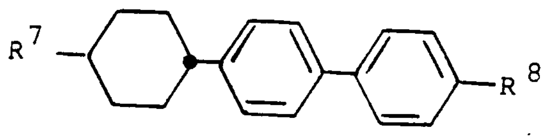

XIII

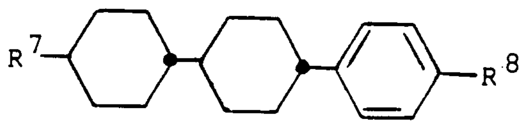

XIV

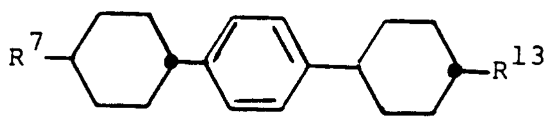

XV

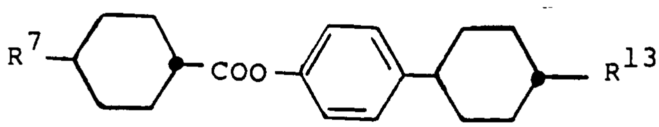

XVI

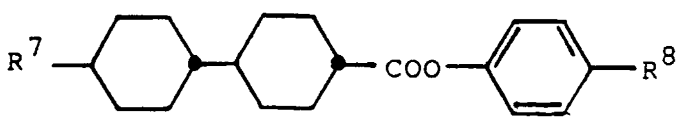

XVII

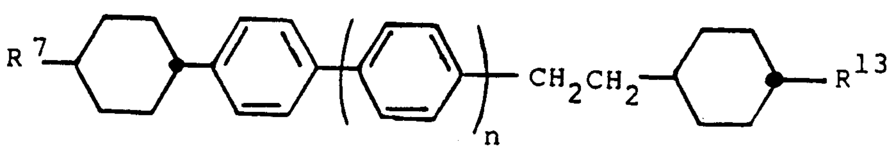

XVIII

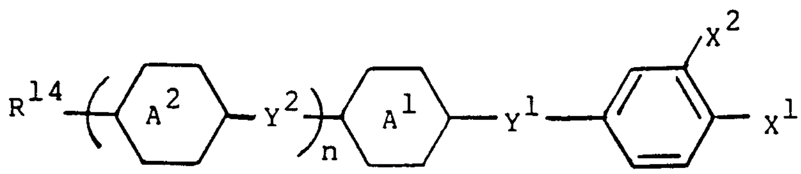

XIX

worin $R^3$ Alkyl, 3E-Alkenyl oder 4-Alkenyl bedeutet; $R^4$ Cyano oder Fluor darstellt; $R^5$ und $R^6$ Alkyl oder Alkoxy bezeichnen; $R^7$ und $R^{13}$ unabhängig voneinander Alkyl, 1E-Alkenyl, 3E-Alkenyl oder 4-Alkenyl bedeuten; $R^8$ Cyano, Alkyl, 1E-Alkenyl, 3E-Alkenyl, 4-Alkenyl, Alkoxy, 2E-Alkenyloxy oder 3-Alkenyloxy bezeichnet; $R^9$ Alkoxy, 2E-Alkenyloxy oder 3-Alkenyloxy bedeutet; n für die Zahl 0 oder 1 steht; Z eine einfache Kovalenzbindung oder $-CH_2CH_2-$ darstellt; $R^{10}$ Cyano, Alkyl, 1E-Alkenyl, 3E-Alkenyl oder 4-Alkenyl bedeutet; $R^{11}$ Alkyl oder 4-Alkenyl bezeichnet; $R^{12}$ Alkoxy, 2E-Alkenyloxy oder 3-Alkenyloxy darstellt; $X^1$ Fluor oder Chlor und $X^2$ Wasserstoff, Fluor oder Chlor bezeichnen; $R^{14}$ Alkyl, 3E-Alkenyl, 4-Alkenyl, Alkoxy, 2E-Alkenyloxy oder 3-Alkenyloxy bedeutet; eine der Gruppen $Y^1$ und $Y^2$ eine einfache Kovalenzbindung, -COO-, -OOC-, $-CH_2CH_2-$, $-CH_2O-$ oder $-OCH_2$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 CH -Gruppen durch Stickstoff ersetzt sind, darstellen.

Der Ausdruck "substituiertes oder unsubstituiertes trans-1,4-Cyclohexylen, in welchem gegebenenfalls 2 nicht benachbarte $CH_2$-Gruppen durch Sauerstoff ersetzt sind", umfasst insbesondere trans-1,4-Cyclohexylen und trans-m-Dioxan-2,5-diyl sowie Ringe, die mit in Flüssigkristallen üblichen Substituenten, wie Cyano, Methyl, Fluor oder Chlor, substituiert sind, beispielsweise 1-Cyano trans-1,4-cyclohexylen oder 2-Methyl-trans-1,4-cyclohexylen.

Der Ausdruck "substituiertes oder unsubstituiertes 1,4-Phenylen, in welchem gegebenenfalls 1 oder 2 CH-Gruppen durch Stickstoff ersetzt sind", umfasst insbesondere 1,4-Phenylen Pyridin-2,5-diyl, Pyrazin-2,5-diyl und Pyrimidin-2,5-diyl sowie Ringe, die mit in Flüssigkristallen üblichen Substituenten, wie Cyano, Methyl, Fluor oder Chlor substituiert sind, beispielsweise 2-Cyano-1,4-phenylen, 2-Fluor-1,4-phenylen, 2-Chlor-1,4-phenylen oder 2-Methyl-1,4-phenylen.

Bevorzugte Mischungskomponenten mit positiver dielektrischer Anisotropie sind die Cyano- und Halogenverbindungen der Formeln II, III, V, VI, VII, IX, XI, XIII, XIV, XVII und XIX. Vorzugsweise enthält das Gesamtgemisch etwa 20-70 Gew. %, insbesondere etwa 25-50 Gew.-% an einer oder mehreren dieser Verbindungen.

Bevorzugte Mischungskomponenten zur Erzielung einer niedrigen optischen Anisotropie im Gesamtgemisch sind die Verbindungen der Formeln V-XIX, insbesondere die Verbindungen der Formeln X, XI, XII, XIV, XV, XVI, XVII und XVIII sowie die Verbindungen der Formel XIX, worin die Ringe $A^1$ und $A^2$ trans-1,4-Cyclohexylen bedeuten.

Besonders bevorzugt sind im allgemeinen diejenigen Gemische, welche neben einer oder mehreren Verbindungen der Formel I eine oder mehrere Verbindungen der Formel V, VII, XI, XII, XIV, XV und/oder XIX enthalten.

Die Verbindungen der Formel XI, worin $R^{10}$ Cyano bedeutet und die Verbindungen der Formel XII worin $R^{11}$ 4-Alkenyl bedeutet, sind neu. Ebenfalls neu sind diejenigen Verbindungen der Formel XIX, worin $R^{14}$ 4-Alkenyl, 2E-Alkenyloxy oder 3-Alkenylox bedeutet, sowie diejenigen, worin $R^{14}$ 3E-Alkenyl bedeutet und $X^2$ Fluor oder Chlor bezeichnet, wenn $X^1$ für Fluor steht. Die Herstellung der neuen Verbindungen der Formeln XI, XII und XIX kann nach den in den Synthesebeispielen veranschaulichten Methoden erfolgen.

Die erfindungsgemässen Gemische können gewünschtenfalls auch dichroitische Farbstoffe enthalten, beispielsweise Azo-, Azoxy- oder Anthrachinonfarbstoffe. Der Anteil an Farbstoffen im Gesamtgemischt beträgt im allgemeinen höchstens etwa 10 Gew.-%.

Die Herstellung der erfindungsgemässen Gemische und die Herstellung der elektro-optischen Vorrichtungen können in an sich bekannter Weise erfolgen.

Die Herstellung der Verbindungen der Formel I und der neuen Verbindungen der Formeln XI, XII und XIX sowie flüssigkristalline Gemische enthaltend diese Verbindungen werden durch die folgenden Beispiele weiter veranschaulicht. C bedeutet eine kristalline, S eine smektische, $S_B$ eine smektisch B, N eine nematische und I die isotrope Phase. $V_{10}$ bezeichnet die Spannung für 10% Transmission (Blickrichtung senkrecht zur Plattenoberfläche). $t_{on}$ und $t_{off}$ bezeichnen die Einschaltzeit bzw. die Ausschaltzeit und $\Delta n$ bezeichnet die optische Anisotropie. $k_{11}$, $k_{22}$ und $k_{33}$ bezeichnen die elastischen Konstanten für Spreizung,

Verdrillung und Biegung. $\Delta\epsilon$ bezeichnet die dielektrische Anisotropie, $\eta$ die Bulkviskosität und $\gamma_1$ die Rotationsviskosität.

Beispiel 1

a) Eine Suspension von 109,6 g 4-(4-Nitrophenyl)cyclohexanon (herstellbar durch Nitrierung von 4-Phenylcyclohexanon) in 1 l Dioxan wurde mit 50 ml Triäthylamin und 2 g 5-prozentiger Palladium-Kohle versetzt und unter gutem Rühren bei Raumtemperatur und 0,3 bar Wasserstoffdruck hydriert. Nach 2 Stunden wurde das Gemisch filtriert. Das Filtrat wurde am Wasserstrahlvakuum bei einer Badtemperatur von 30 °C eingedampft und der Eindampfrückstand über Nacht im Trockenschrank am Wasserstrahlvakuum bei 40 °C getrocknet. Hierbei wurden 94,5 g 4-(4-Aminophenyl)cyclohexanon als weisse Kristalle mit Smp. 127-128 °C erhalten.

b) In einem Sulfierkolben wurden 200 ml 4N Schwefelsäure auf 80 °C erwärmt und dann mit ca. 5% einer Lösung von 37,9 g 4-(4-Aminophenyl)cyclohexanon in 200 ml 4N Schwefelsäure versetzt. Anschliessend wurde bei 80 °C innert 1,5 Stunden gleichzeitig die restliche Lösung von 4-(4-Aminophenyl)-cyclohexanon sowie eine Lösung von 15,2 g Natriumnitrit in 45 ml Wasser zum Reaktionsgemisch zugetropft. Danach wurde das Gemisch bei 80 °C innert 30 Minuten tropfenweise mit einer Lösung von 9 g Natriumnitrit in 27 ml Wasser versetzt und noch 1 Stunde bei 80 °C weitergerührt. Nach dem Abkühlen des Reaktionsgemisches auf 0 °C wurden die ausgefallenen Kristalle abgenutscht, mit 200 ml kaltem Wasser gewaschen und im Trockenschrank am Wasserstrahlvakuum bei 60 °C bis zur Gewichtskonstanz getrocknet. Das kristalline Rohprodukt (34.6 g) wurde in 520 ml Aethylacetat suspendiert. Die Suspension wurde 1 Stunde zum Rückfluss erhitzt, dann mit 1.7 g Aktivkohle versetzt und dann noch 1 Stunde zum Rückfluss erhitzt. Anschliessend wurde das Gemisch genutscht (Nachwaschen mit 40 ml warmem Aethylacetat) und das Filtrat am Wasserstrahlvakuum bei einer Badtemperatur von 40 °C eingedampft. Trocknung des Eindampfrückstandes im Trockenschrank am Wasserstrahlvakuum bei 60 °C bis zur Gewichtskonstanz ergab 32,2 g 4-(4-Hydroxyphenyl)cyclohexanon als gelb-braune Kristalle mit Smp. 165-166 °C.

c) In einem Sulfierkolben wurde unter schwachem Stickstoffstrom eine Suspension von 3,8 g 4-(4-Hydroxyphenyl)cyclohexanon in 60 ml tert.Butylmethyläther mit 10,6 g 2-(1,3-Dioxolan-2-yl)äthyl-triphenylphosphoniumbromid und 2,2 g Kalium-tert.butylat versetzt. Das Gemisch wurde zunächst 30 Minuten gerührt, dann innert 2,25 Stunden bei 25 °C portionenweise mit weiteren 2,7 g Kalium-tert.butylat versetzt und noch 1 Stunde bei Raumtemperatur gerührt. Danach wurde das Reaktionsgemisch mit weiteren 1,8 2-(1,3-Dioxolan-2-yl)äthyl-triphenylphosphoniumbromid und 0,45 g Kalium-tert.butylat versetzt und noch 1 Stunde gerührt. Anschliessend wurde das Reaktionsgemisch auf 80 ml Wasser gegossen und mit 11 ml 2N Schwefelsäure angesäuert. Die wässrige Phase wurde abgetrennt und zweimal mit je 80 ml tert.Butylmethyläther extrahiert. Die organischen Phasen wurden zweimal mit je 50 ml Wasser gewaschen, über Natriumsulfat getrocknet und genutscht. Das Filtrat wurde am Wasserstrahlvakuum bei einer Badtemperatur von 40 °C bis zur Gewichtskonstanz eingedampft. Das erhaltene rötliche Oel (10,9 g) wurde an Kieselgel mit Toluol und Toluol/tert.-Butylmethyläther (Vol. 20:1) chromatographisch getrennt. Eindampfen der Produktfraktionen am Wasserstrahlvakuum und Trocknung des Rückstandes im Trockenschrank am Wasserstrahlvakuum bei 60 °C bis zur Gewichtskonstanz ergab schliesslich 4,5 g 4-[4-[2-(1,3-Dioxolan-2-yl)äthyliden]cyclohexyl]phenol als gelbliche Kristalle mit Smp. 125,5 126.5 °C.

d) Eine Lösung von 36,5 g 4-[4-[2-(1,3-Dioxolan-2-yl)äthyliden]cyclohexyl]phenol in 500 ml Toluol und 50 ml Triäthylamin wurde in einem Hydrierautoklaven mit 3,2 g 5-prozentiger Platin-Kohle und 10 bar Wasserstoff bei 90 °C 1 Stunde hydriert. Anschliessend wurde das Gemisch genutscht und der Rückstand mit 30 ml warmem Toluol gewaschen. Das Filtrat wurde am Wasserstrahlvakuum bei einer Badtemperatur von 40 °C bis zur Gewichtskonstanz eingedampft. Die erhaltenen weissen Kristalle (37 g) wurden in 180 ml Methanol heiss gelöst. Die Lösung wurde auf Raumtemperatur abkühlen gelassen und dann 5 Stunden in den Kühlschrank gestellt. Anschliessend wurden die Kristalle abgenutscht, mit 50 ml kaltem Methanol gewaschen und im Trockenschrank am Wasserstrahlvakuum bei 60 °C getrocknet. Hierbei wurden 27,6 g 4-[trans-4-[2-(1,3-Dioxolan-2-yl)äthyl]cyclohexyl]phenol als weisse Kristalle mit Smp. 153,5-154,5 °C erhalten.

e) In einem Stahl-Rührautoklaven wurde ein Gemisch von 100 g 4-[trans-4-[2-(1,3-Dioxolan-2-yl)äthyl]-cyclohexyl]phenol, 10 g 5-prozentigem Rhodium/Aluminiumoxid und 1 l Aethylacetat mit 50 bar Wasserstoff bei 80 °C 90 Minuten hydriert. Dann wurde der Katalysator abgenutscht und mit 100 ml Aethylacetat gewaschen. Das Filtrat wurde eingedampft und der Rückstand bei 25 °C/0,4 mbar getrocknet. Hierbei wurden 101.75 g 4-[trans-4-[2-(1,3-Dioxolan-2-yl)äthyl]cyclohexyl]cyclohexanol enthaltend 59% cis-Isomer und 36% trans-Isomer erhalten.

f) Eine Lösung von 12,9 g Pyridiniumchlorochromat in 80 ml Methylenchlorid wurde bei Raumtemperatur unter Stickstoffbegasung innert 5 Minuten tropfenweise mit einer Lösung von 13,0 g 4-[trans-4-[2-(1,3-Dioxolan-2-yl)äthyl]cyclohexyl]cyclohexanol in 40 ml Methylenchlorid versetzt. Das Gemisch wurde noch 1 Stunde gerührt, dann mit 100 ml Diäthyläther verdünnt und filtriert und das Filtrat eingedampft. Der Eindampfrückstand wurde in 200 ml Diäthyläther aufgenommen, das Gemisch filtriert, das Filtrat eingedampft und dann dieses Vorgehen noch zweimal wiederholt. Danach wurde die erhaltene, bräunliche feste Masse (12,1 g) an Kieselgel mit Aethylacetat/Petroläther (Vol. 1:4) chromatographisch getrennt. Kristallisation der farblosen Ketonfraktion aus 60 ml Aethylacetat und 200 ml Petroläther ergab schliesslich 7,0 g reines 4-[trans-4 -[2-(1,3-Dioxolan-2-yl)äthyl]cyclohexyl]cyclohexanon; Smp. 100,4 °C.

g) Eine Suspension von 2,97 g Natriumborhydrid in 300 ml Isopropanol wurde unter Stickstoffbegasung bei -70 °C tropfenweise mit einer Lösung von 11 g 4-[trans-4-[2-(1,3-Dioxolan-2-yl)äthyl]cyclohexyl]-cyclohexanon in 200 ml Isopropanol versetzt. Nach ca. 1 Stunde wurde das Reaktionsgemisch auf Raumtemperatur erwärmen gelassen, mit 500 ml 0,1N Salzsäure verdünnt und dreimal mit je 300 ml Methylenchlorid extrahiert. Die organischen Phasen wurden mit Wasser gewaschen, über Magnesiumsulfat getrocknet, filtriert und eingedampft. Kristallisation des Rückstandes (11 g) aus 500 ml Aethylacetat/Petroläther (Vol. 3:5) ergab 6,6 g reines trans-4-[trans-4 -[2-(1,3-Dioxolan-2-yl)äthyl]-cyclohexyl]cyclohexanol mit Smp. 129,5 °C.

h) In einem Rundkolben wurden unter Stickstoffbegasung 2.04 g Natriumhydrid als ca. 50-prozentige ölige Suspension vorgelegt und zweimal mit Pentan gewaschen. Dann wurden zum Natriumhydrid 40 ml trockenes Tetrahydrofuran und eine Lösung von 6,0 g trans-4-[trans-4 -[2-(1,3-Dioxolan-2-yl)äthyl]-cyclohexyl]cyclohexanol in 30 ml Tetrahydrofuran zugefügt. Das Gemisch wurde bei Raumtemperatur 30 Minuten gerührt, dann mit 4,0 ml Methyljodid versetzt und 2 Stunden zum Rückfluss erhitzt. Anschliessend wurde das Reaktionsgemisch abgekühlt, in 200 ml Diäthyläther aufgenommen und dreimal mit je 200 ml Wasser gewaschen. Die organische Phase wurde über Magnesiumsulfat getrocknet, filtriert und eingedampft, wobei 6,3 g 2-[2-[trans-4-(trans -4-Methoxycyclohexyl)cyclohexyl]äthyl]-1,3-dioxolan mit Smp. 74 °C erhalten wurden.

i) 6,2 g 2-[2-[trans-4-(trans-4-Methoxycyclohexyl)cyclohexyl]äthyl] -1,3-dioxolan wurden unter Stickstoffbegasung mit 100 ml Wasser, 50 ml Eisessig und 20 ml Dioxan versetzt. Das Gemisch wurde 1,5 Stunden bei 100 °C (Badtemperatur) gerührt, dann mit verdünnter Natriumhydrogencarbonat-Lösung neutralisiert und dreimal mit Diäthyläther extrahiert. Die vereinigten Aetherphasen wurden einmal mit Wasser und zweimal mit verdünnter Natriumhydrogencarbonat-Lösung gewaschen, über Magnesiumsulfat getrocknet, filtriert und eingedampft. Umkristallisation des erhaltenen rohen Aldehyds (4,34 g) aus 150 ml Petroläther bei -20 °C ergab 2,8 g 3-[trans-4-(trans -4-Methoxycyclohexyl)cyclohexyl]propionaldehyd in einer Reinheit von 96%; Smp. 36 °C.

j) 2,65 g Aethyltriphenylphosphoniumbromid wurden unter Argonbegasung in 40 ml tert.Butylmethyläther aufgeschlämmt. Die Suspension wurde bei Raumtemperatur mit 797 mg Kalium-tert.butylat versetzt und 1 Stunde gerührt. Anschliessend wurde das Gemisch auf 0 °C gekühlt, innert 3 Minuten tropfenweise mit einer Lösung von 1,1 g 3-[trans-4-(trans-4-Methoxycyclohexyl)cyclohexyl]propionaldehyd in 15 ml tert.Butylmethyläther versetzt und dann unter Rühren langsam auf Raumtemperatur erwärmen gelassen. Nach 2 Stunden wurde die hellgelbe Suspension in Diäthyläther/Wasser verteilt. Die wässrige Phase wurde abgetrennt und dreimal mit Diäthyläther gewaschen. Die organischen Phasen wurden zweimal mit Wasser gewaschen, über Magnesiumsulfat getrocknet, filtriert und eingedampft. Chromatographische Reinigung des gelben, festen Rohproduktes an Kieselgel mit Aethylacetat/Petroläther (Vol. 3:97) ergab 0,91 g trans-4-(3-Pentenyl)-1-(trans-4-methoxycyclohexyl)cyclohexan mit einem Z/E-Verhältnis von 86:11.

k) 0,91 g trans-4-(3-Pentenyl)-1-(trans-4-methoxycyclohexyl)cyclohexan (Z/E = 86:11) wurden unter Stickstoffbegasung mit 6 ml Toluol, 0,11 g Natriumbenzolsulfinat und 1 ml 1N Salzsäure versetzt. Das Gemisch wurde 15 Stunden bei 50 °C gerührt, dann auf 100 ml verdünnte Natriumhydrogencarbonat-Lösung gegossen und dreimal mit je 50 ml Diäthyläther extrahiert. Die vereinigten organischen Phasen wurden mit 100 ml verdünnter Natriumcarbonat-Lösung und mit 100 ml Wasser gewaschen, über Magnesiumsulfat getrocknet, filtriert und eingedampft. Das erhaltene gelbliche Oel (0,9 g) wurde an mit Silbernitrat imprägniertem Kieselgel mit Diäthyläther/Hexan (Vol. 1:9) chromatographisch gereinigt. Umkristallisation des erhaltenen Produktes (486 mg) aus 10 ml Methanol bei -20 °C ergab reines trans-4-(3E-Pentenyl)-1-(trans-4-methoxycyclohexyl)cyclohexan mit Smp. (C-N) 16,6 °C und Klp. (N-I) 43,7 °C.

In analoger Weise können folgende Verbindungen hergestellt werden:

trans-4-(3-Butenyl)-1-(trans-4-methoxycyclohexyl)cyclohexan, Smp. (C-N) -13,6 °C, Klp. (N-I) 18,0 °C;
trans-4-(3-Butenyl)-1-(trans-4-äthoxycyclohexyl)cyclohexan, Smp. (C-N) 13,1 °C, Klp. (N-I) 45,3 °C;
trans-4-(3-Butenyl)-1-(trans-4-propyloxycyclohexyl)cyclohexan;

trans-4-(3-Butenyl)-1-(trans-4-butyloxycyclohexyl)cyclohexan;
trans-4-(3-Butenyl)-1-(trans-4-pentyloxycyclohexyl)cyclohexan;
trans-4-(3-Butenyl)-1-(trans-4-hexyloxycyclohexyl)cyclohexan;
trans-4-(3-Butenyl)-1-(trans-4-allyloxycyclohexyl)cyclohexan;
trans-4-(3-Butenyl)-1-[trans-4-(2E-butenyloxy)cyclohexyl]cyclohexan,
trans-4-(3-Butenyl)-1-[trans-4-(2E-pentenyloxy)cyclohexyl]cyclohexan,
trans-4-(3-Butenyl)-1-[trans-4-(2E-hexenyloxy)cyclohexyl]cyclohexan,
trans-4-(3-Butenyl)-1-[trans-4-(3-butenyloxy)cyclohexyl]cyclohexan,
trans-4-(3-Butenyl)-1-[trans-4-(3Z-pentenyloxy)cyclohexyl]cyclohexan,
trans-4-(3-Butenyl)-1-[trans-4-(3Z-hexenyloxy)cyclohexyl]cyclohexan,
trans-4-(3E-Pentenyl)-1-(trans-4-äthoxycyclohexyl)cyclohexan, Smp. (C-N) 44,5 °C, Klp. (N-I) 76,5 °C;
trans-4-(3E-Pentenyl)-1-(trans-4-propyloxycyclohexyl)cyclohexan;
trans-4-(3E-Pentenyl)-1-(trans-4-butyloxycyclohexyl)cyclohexan;
trans-4-(3E-Pentenyl)-1-(trans-4-pentyloxycyclohexyl)cyclohexan;
trans-4-(3E-Pentenyl)-1-(trans-4-hexyloxycyclohexyl)cyclohexan;
trans-4-(3E-Pentenyl)-1-(trans-4-allyloxycyclohexyl)cyclohexan;
trans-4-(3E-Pentenyl)-1-[trans-4-(2E-butenyloxy)cyclohexyl]cyclohexan;
trans-4-(3E-Pentenyl)-1-[trans-4-(2E-pentenyloxy)cyclohexyl]cyclohexan;
trans-4-(3E-Pentenyl)-1-[trans-4-(2E-hexenyloxy)cyclohexyl]cyclohexan;
trans-4-(3E-Pentenyl)-1-[trans-4-(3-butenyloxy)cyclohexyl]cyclohexan;
trans-4-(3E-Pentenyl)-1-[trans-4-(3Z-pentenyloxy)cyclohexyl]cyclohexan;
trans-4-(3E-Pentenyl)-1-[trans-4-(3Z-hexenyloxy)cyclohexyl]cyclohexan;
trans-4-(3E-Hexenyl)-1-(trans-4-methoxycyclohexyl)cyclohexan;
trans-4-(3E-Hexenyl)-1-(trans-4-äthoxycyclohexyl)cyclohexan;
trans-4-(3E-Hexenyl)-1-(trans-4-propyloxycyclohexyl)cyclohexan;
trans-4-(3E-Hexenyl)-1-(trans-4-butyloxycyclohexyl)cyclohexan;
trans-4-(3E-Hexenyl)-1-(trans-4-pentyloxycyclohexyl)cyclohexan;
trans-4-(3E-Hexenyl)-1-(trans-4-hexyloxycyclohexyl)cyclohexan;
trans-4-(3E-Hexenyl)-1-(trans-4-allyloxycyclohexyl)cyclohexan;
trans-4-(3E-Hexenyl)-1-[trans-4-(2E-butenyloxy)cyclohexyl]cylohexan;
trans-4-(3E-Hexenyl)-1-[trans-4-(2E-pentenyloxy)cyclohexyl]cylohexan;
trans-4-(3E-Hexenyl)-1-[trans-4-(2E-hexenyloxy)cyclohexyl]cylohexan;
trans-4-(3E-Hexenyl)-1-[trans-4-(3-butenyloxy)cyclohexyl]cylohexan;
trans-4-(3E-Hexenyl)-1-[trans-4-(3Z-pentenyloxy)cyclohexyl]cylohexan;
trans-4-(3E-Hexenyl)-1-[trans-4-(3Z-hexenyloxy)cyclohexyl]cylohexan.

In analoger Weise zu den Stufen a) bis i) und weitere Umsetzung des erhaltenen Propionaldehyds in analoger Weise zu Beispiel 2, Stufen h), i) und j) können folgende Verbindungen hergestellt werden:

trans-4-(4-Pentenyl)-1-(trans-4-methoxycyclohexyl)cyclohexan, Smp. (C-$S_B$) 7,7 °C, Klp. ($S_B$-I) 14,0 °C;
trans-4-(4-Pentenyl)-1-(trans-4-äthoxycyclohexyl)cyclohexan, Smp. (C-$S_B$) 10,5 °C, Klp. ($S_B$-I) 43,1 °C:
trans-4-(4-Pentenyl)-1-(trans-4-propyloxycyclohexyl)cyclohexan;
trans-4-(4-Pentenyl)-1-(trans-4-butyloxycyclohexyl)cyclohexan;
trans-4-(4-Pentenyl)-1-(trans-4-pentyloxycyclohexyl)cyclohexan;
trans-4-(4-Pentenyl)-1-(trans-4-hexyloxycyclohexyl)cyclohexan;
trans-4-(4-Pentenyl)-1-(trans-4-allyloxycyclohexyl)cyclohexan;
trans-4-(4-Pentenyl)-1-[trans-4-(3-butenyloxy)cyclohexyl]cyclohexan;
trans-4-(4Z-Hexenyl)-1-(trans-4-methoxycyclohexyl)cyclohexan;
trans-4-(4Z-Hexenyl)-1-(trans-4-äthoxycyclohexyl)cyclohexan;
trans-4-(4Z-Hexenyl)-1-(trans-4-propyloxycyclohexyl)cyclohexan;
trans-4-(4Z-Hexenyl)-1-(trans-4-butyloxycyclohexyl)cyclohexan;
trans-4-(4Z-Hexenyl)-1-(trans-4-pentyloxycyclohexyl)cyclohexan;
trans-4-(4Z-Hexenyl)-1-(trans-4-hexyloxycyclohexyl)cyclohexan;
trans-4-(4Z-Hexenyl)-1-(trans-4-allyloxycyclohexyl)cyclohexan;
trans-4-(4Z-Hexenyl)-1-[trans-4-(3-butenyloxy)cyclohexyl]cyclohexan.

Durch Umsetzung des 4-(4-Hydroxyphenyl)cyclohexanons (Stufe b) in analoger Weise zu Beispiel 2, Stufen h) und i) Acetalisierung des erhaltenen 4-(trans-4-Formylcyclohexyl)phenols mit Aethylenglykol in Gegenwart von p-Toluolsulfonsäure und weitere Umsetzung des Dioxolans in analoger Weise zu den vorliegenden Stufen e) bis k) können folgende Verbindungen hergestellt werden:

trans-4-Vinyl-1-(trans-4-methoxycyclohexyl)cyclohexan, Smp. (C-I) -20,3 °C, Klp. (N-1) -26,5 °C;

trans-4-Vinyl-1-(trans-4-äthoxycyclohexyl)cyclohexan, Smp. (C-N) -17 °C, Klp.(N-I) 6,3 °C;
trans-4-Vinyl-1-(trans-4-propyloxycyclohexyl)cyclohexan;
trans-4-Vinyl-1-(trans-4-butyloxycyclohexyl)cyclohexan;
trans-4-Vinyl-1-(trans-4-pentyloxycyclohexyl)cyclohexan;
trans-4-Vinyl-1-(trans-4-hexyloxycyclohexyl)cyclohexan;
trans-4-Vinyl-1-(trans-4-allyloxycyclohexyl)cyclohexan;
trans-4-Vinyl-1-[trans-4-(3-butenyloxy)cyclohexyl]cyclohexan;
trans-4-(1E-propenyl)-1-(trans-4-methoxycyclohexyl)cyclohexan, Smp. (C-N) 4,2 °C, Klp. (N-I) 17,2 °C;
trans-4-(1E-Propenyl)-1-(trans-4-äthoxycyclohexyl)cyclohexan. Smp. (C-$S_B$) 37 °C, Uebergang ($S_B$-N) 37,5 °C, Klp. (N-I) 67,6 °C;
trans-4-(1E-Propenyl)-1-(trans-4-propyloxycyclohexyl)cyclohexan;
trans-4-(1E-Propenyl)-1-(trans-4-butyloxycyclohexyl)cyclohexan;
trans-4-(1E-Propenyl)-1-(trans-4-pentyloxycyclohexyl)cyclohexan;
trans-4-(1E-Propenyl)-1-(trans-4-hexyloxycyclohexyl)cyclohexan;
trans-4-(1E-Propenyl)-1-(trans-4-allyloxycyclohexyl)cyclohexan;
trans-4-(1E-Propenyl)-1-[trans-4-(3-butenyloxy)cyclohexyl]cyclohexan;
trans-4-(1E-Butenyl)-1-(trans-4-methoxycyclohexyl)cyclohexan, Smp. (C-$S_B$) -2,9 °C, Uebergang ($S_B$-N) 3,4 °C, Klp. (N-I) 8,4 °C;
trans-4-(1E-Butenyl)-1-(trans-4-äthoxycyclohexyl)cyclohexan. Smp. (C-$S_B$) 8,3 °C, Uebergang ($S_B$-N) 21.5 °C, Klp. (N-I) 49.7 °C;
trans-4-(1E-Butenyl)-1-(trans-4-propyloxycyclohexyl)cyclohexan;
trans-4-(1E-Butenyl)-1-(trans-4-butyloxycyclohexyl)cyclohexan;
trans-4-(1E-Butenyl)-1-(trans-4-pentyloxycyclohexyl)cyclohexan;
trans-4-(1E-Butenyl)-1-(trans-4-allyloxycyclohexyl)cyclohexan;
trans-4-(1E-Butenyl)-1-[trans-4-(3-butenyloxy)cyclohexyl]cyclohexan;
trans-4-(1E-Pentenyl)-1-(trans-4-methoxycyclohexyl)cyclohexan, Smp. (C-$S_B$) -17,4 °C, Uebergang ($S_B$-N) 21,5 °C, Klp. (N-I) 24,7 °C;
trans-4-(1E-Pentenyl)-1-(trans-4-äthoxycyclohexyl)cyclohexan, Smp. (C-$S_B$) 14 °C, Uebergang ($S_B$-N) 48,1 °C, Klp. (N-I) 62,5 °C;
trans-4-(1E-Pentenyl)-1-(trans-4-propyloxycyclohexyl)cyclohexan;
trans-4-(1E-Pentenyl)-1-(trans-4-butyloxycyclohexyl)cyclohexan;
trans-4-(1E-Pentenyl)-1-(trans-4-allyloxycyclohexyl)cyclohexan;
trans-4-(1E-Pentenyl)-1-[trans-4-(3-butenyloxy)cyclohexyl]cyclohexan.

Ausgehend von 4-(2-Phenyläthyl)cyclohexanon [herstellbar in analoger Weise zum in Mol. Cryst. Liq. Cryst. 131, 327 (1985) beschriebenen Nitril] können nach der obigen Methode auch die folgenden Verbindungen hergestellt werden:

4-[2-[trans-4-(2-(1,3-Dioxolan-2-yl)äthyl)cyclohexyl]äthyl]cyclohexanon;
trans-4-(3-Butenyl)-1-[2-(trans-4-methoxycyclohexyl)äthyl]cyclohexan;
trans-4-(3-Butenyl)-1-[2-(trans-4-äthoxycyclohexyl)äthyl]cyclohexan;
trans-4-(3-Butenyl)-1-[2-(trans-4-propyloxycyclohexyl)äthyl]cyclohexan;
trans-4-(3-Butenyl)-1-[2-(trans-4-butyloxycyclohexyl)äthyl]cyclohexan;
trans-4-(3-Butenyl)-1-[2-(trans-4-pentyloxycyclohexyl)äthyl]cyclohexan;
trans-4-(3-Butenyl)-1-[2-(trans-4-hexyloxycyclohexyl)äthyl]cyclohexan;
trans-4-(3-Butenyl)-1-[2-(trans-4-allyloxycyclohexyl)äthyl]cyclohexan;
trans-4-(3-Butenyl)-1-[2-(trans-4-(2E-butenyloxy)cyclohexyl)äthyl]cyclohexan;
trans-4-(3-Butenyl)-1-[2-(trans-4-(2E-pentenyloxy)cyclohexyl)äthyl]cyclohexan;
trans-4-(3-Butenyl)-1-[2-(trans-4-(2E-hexenyloxy)cyclohexyl)äthyl]cyclohexan;
trans-4-(3-Butenyl)-1-[2-(trans-4-(3-butenyloxy)cyclohexyl)äthyl]cyclohexan;
trans-4-(3-Butenyl)-1-[2-(trans-4-(3Z-pentenyloxy)cyclohexyl)äthyl]cyclohexan;
trans-4-(3-Butenyl)-1-[2-(trans-4-(3Z-hexenyloxy)cyclohexyl)äthyl]cyclohexan;
trans-4-(3E-Pentenyl)-1-[2-(trans-4-methoxycyclohexyl)äthyl]cyclohexan;
trans-4-(3E-Pentenyl)-1-[2-(trans-4-äthoxycyclohexyl)äthyl]cyclohexan;
trans-4-(3E-Pentenyl)-1-[2-(trans-4-propyloxycyclohexyl)äthyl]cyclohexan;
trans-4-(3E-Pentenyl)-1-[2-(trans-4-butyloxycyclohexyl)äthyl]cyclohexan;
trans-4-(3E-Pentenyl)-1-[2-(trans-4-pentyloxycyclohexyl)äthyl]cyclohexan;
trans-4-(3E-Pentenyl)-1-[2-(trans-4-hexyloxycyclohexyl)äthyl]cyclohexan;
trans-4-(3E-Pentenyl)-1-[2-(trans-4-allyloxycyclohexyl)äthyl]cyclohexan;
trans-4-(3E-Pentenyl)-1-[2-(trans-4-(2E-butenyloxy)cyclohexyl)äthyl]cyclohexan;

trans-4-(3E-Pentenyl)-1-[2-(trans-4-(2E-pentenyloxy)cyclohexyl)äthyl]cyclohexan;
trans-4-(3E-Pentenyl)-1-[2-(trans-4-(2E-hexenyloxy)cyclohexyl)äthyl]cyclohexan;
trans-4-(3E-Pentenyl)-1-[2-(trans-4-(3-butenyloxy)cyclohexyl)äthyl]cyclohexan;
trans-4-(3E-Pentenyl)-1-[2-(trans-4-(3Z-pentenyloxy)cyclohexyl)äthyl]cyclohexan;
trans-4-(3E-Pentenyl)-1-[2-(trans-4-(3Z-hexenyloxy)cyclohexyl)äthyl]cyclohexan;
trans-4-(3E-Hexenyl)-1-[2-(trans-4-methoxycyclohexyl)äthyl]cyclohexan;
trans-4-(3E-Hexenyl)-1-[2-(trans-4-äthoxycyclohexyl) äthyl]cyclohexan;
trans-4-(3E-Hexenyl)-1-[2-(trans-4-propyloxycyclohexyl)äthyl]cyclohexan;
trans-4-(3E-Hexenyl)-1-[2-(trans-4-butyloxycyclohexyl)äthyl]cyclohexan;
trans-4-(3E-Hexenyl) 1-[2-(trans-4-pentyloxycyclohexyl)äthyl]cyclohexan;
trans-4-(3E-Hexenyl)-1-[2-(trans-4-hexyloxycyclohexyl)äthyl]cyclohexan;
trans-4-(3E-Hexenyl)-1-[2-(trans-4-allyloxycyclohexyl)äthyl]cyclohexan;
trans-4-(3E-Hexenyl)-1-[2-(trans-4-(3-butenyloxy)cyclohexyl)äthyl]cyclohexan;
trans-4-(4-Pentenyl)-1-[2-(trans-4-methoxycyclohexyl)äthyl]cyclohexan;
trans-4-(4-Pentenyl)-1-[2-(trans-4-äthoxycyclohexyl)äthyl]cyclohexan;
trans-4-(4-Pentenyl)-1-[2-(trans-4-propyloxycyclohexyl)äthyl]cyclohexan;
trans-4-(4-Pentenyl)-1-[2-(trans-4-butyloxycyclohexyl)äthyl]cyclohexan;
trans-4-(4-Pentenyl)-1-[2-(trans-4-pentyloxycyclohexyl)äthyl]cyclohexan;
trans-4-(4-Pentenyl)-1-[2-(trans-4-hexyloxycyclohexyl)äthyl]cyclohexan;
trans-4-(4-Pentenyl)-1-[2-(trans-4-allyloxycyclohexyl)äthyl]cyclohexan;
trans-4-(4-Pentenyl)-1-[2-(trans-4-(3-butenyloxy)cyclohexyl)äthyl]cyclohexan;
trans-4-(4Z-Hexenyl)-1-[2-(trans-4-methoxycyclohexyl)äthyl]cyclohexan;
trans-4-(4Z-Hexenyl)-1-[2-(trans-4-äthoxycyclohexyl)äthyl]cyclohexan;
trans-4-(4Z-Hexenyl)-1-[2-(trans-4-propyloxycyclohexyl)äthyl]cyclohexan;
trans-4-(4Z-Hexenyl)-1-[2-(trans-4-butyloxycyclohexyl)äthyl]cyclohexan;
trans-4-(4Z-Hexenyl)-1-[2-(trans-4-allyloxycyclohexyl)äthyl]cyclohexan;
trans-4-(4Z-Hexenyl)-1-[2-(trans-4-(3-butenyloxy)cyclohexyl)äthyl]cyclohexan;
trans-4-Vinyl-1-[2-(trans-4-methoxycyclohexyl)äthyl]cyclohexan;
trans-4-Vinyl-1-[2-(trans-4-äthoxycyclohexyl)äthyl]cyclohexan;
trans-4-Vinyl-1-[2-(trans-4-propyloxycyclohexyl)äthyl]cyclohexan;
trans-4-Vinyl-1-[2-(trans-4-butyloxycyclohexyl)äthyl]cyclohexan;
trans-4-Vinyl-1-[2-(trans-4-pentyloxycyclohexyl)äthyl]cyclohexan;
trans-4-Vinyl-1-[2-(trans-4-hexyloxycyclohexyl)äthyl]cyclohexan;
trans-4-Vinyl-1-[2-(trans-4-allyloxycyclohexyl)äthyl]cyclohexan;
trans-4-Vinyl-1-[2-(trans-4-(3-butenyloxy)cyclohexyl)äthyl]cyclohexan;
trans-4-(1E-Propenyl)-1-[2-(trans-4-methoxycyclohexyl)äthyl]cyclohexan;
trans-4-(1E-Propenyl)-1-[2-(trans-4-äthoxycyclohexyl)äthyl]cyclohexan;
trans-4-(1E-Propenyl)-1-[2-(trans-4-propyloxycyclohexyl)äthyl]cyclohexan;
trans-4-(1E-Propenyl)-1-[2-(trans-4-butyloxycyclohexyl)äthyl]cyclohexan;
trans-4-(1E-Propenyl)-1-[2-(trans-4-pentyloxycyclohexyl)äthyl]cyclohexan;
trans-4-(1E-Propenyl)-1-[2-(trans-4-hexyloxycyclohexyl)äthyl]cyclohexan;
trans-4-(1E-Propenyl)-1-[2-(trans-4-allyloxycyclohexyl)äthyl]cyclohexan;
trans-4-(1E-Propenyl)-1-[2-(trans-4-(3-butenyloxy)cyclohexyl)äthyl]cyclohexan;
trans-4-(1E-Butenyl)-1-[2-(trans-4-methoxycyclohexyl)äthyl]cyclohexan;
trans-4-(1E-Butenyl)-1-[2-(trans-4-äthoxycyclohexyl)äthyl]cyclohexan;
trans-4-(1E-Butenyl)-1-[2-(trans-4-propyloxycyclohexyl)äthyl]cyclohexan;
trans-4-(1E-Butenyl)-1-[2-(trans-4-butyloxycyclohexyl)äthyl]cyclohexan;
trans-4-(1E-Butenyl)-1-[2-(trans-4-pentyloxycyclohexyl)äthyl]cyclohexan;
trans-4-(1E-Butenyl)-1-[2-(trans-4-allyloxycyclohexyl)äthyl]cyclohexan;
trans-4-(1E-Butenyl)-1-[2-(trans-4-(3-butenyloxy)cyclohexyl)äthyl]cyclohexan;
trans-4-(1E-Pentenyl)-1-[2-(trans-4-methoxycyclohexyl)äthyl]cyclohexan;
trans-4-(1E-Pentenyl)-1-[2-(trans-4-äthoxycyclohexyl)äthyl]cyclohexan;
trans-4-(1E-Pentenyl)-1-[2-(trans-4-propyloxycyclohexyl)äthyl]cyclohexan;
trans-4-(1E-Pentenyl)-1-[2-(trans-4-butyloxycyclohexyl)äthyl]cyclohexan;
trans-4-(1E-Pentenyl)-1-[2-(trans-4-allyloxycyclohexyl)äthyl]cyclohexan;
trans-4-(1E-Pentenyl)-1-[2-(trans-4-(3-butenyloxy)cyclohexyl)äthyl]cyclohexan.

Beispiel 2

a) 140,9 g 2-(1,3-Dioxolan-2-yl)äthyl-triphenylphosphoniumbromid wurden unter Stickstoffbegasung in 4,5 l Tetrahydrofuran suspendiert und die Suspension bei 0 °C innert 5 Minuten mit 36,8 g Kalium-tert.butylat versetzt. Die orange Suspension wurde noch 1 Stunde bei Raumtemperatur gerührt und innert 5 Minuten sit 46,1 g 4-(4-Oxocyclohexyl)benzamid versetzt. Das Reaktionsgemisch wurde noch 4,5 Stunden bei Raumtemperatur gerührt und dann im Vakuum eingeengt. Die erhaltenen, gelblichen Kristalle (212,5 g) wurden mit 1,2 l Diäthyläther versetzt. Das Gemisch wurde 30 Minuten bei Raumtemperatur gerührt und dann genutscht. Der Rückstand wurde mit Diäthyläther gewaschen und dann in 700 ml Wasser aufgeschlämmt. Das Gemisch wurde 15 Minuten gerührt und dann genutscht. Der Rückstand wurde mit Wasser gewaschen und in 600 ml Dioxan heiss gelöst. Die Lösung wurde auf Raumtemperatur abkühlen gelassen. Die ausgefallenen Kristalle wurden abgenutscht, mit wenig Dioxan und Hexan gewaschen und im Vakuum bei 60 °C getrocknet, wobei 39,2 g kristallines 4-[4-[2-(1,3-Dioxolan-2-yl)-äthyliden]cyclohexyl]benzamid mit Smp. 209-212 °C erhalten wurden. Aufarbeitung der Mutterlauge (72,6 g) ergab weitere 7,7 g Produkt und 64,2 g zweite Mutterlauge.

b) Ein Gemisch von 500 mg 4-[4-[2-(1,3-Dioxolan-2-yl)äthyliden]cyclohexyl]benzamid und 20 ml Dioxan/Triäthylamin (Vol. 9:1) wurde mittels 500 mg 10-prozentiger platin-Kohle 2 Stunden hydriert. Dann wurde das Reaktionsgemisch filtriert und das Filtrat eingedampft. Umkristallisation des Eindampfrückstandes aus 40 ml Dioxan ergab 230 mg 4-[trans-4-[2-(1,3-Dioxolan-2-yl)äthyl]cyclohexyl]benzamid als farblose Kristalle.

c) 27 g 4-[trans-4-[2-(1,3-Dioxolan-2-yl)äthyl]cyclohexyl]benzamid wurden mit 5 g 5-prozentiger Ruthenium Kohle in Dioxan bei 120 °C und 40 bar Wasserstoff 8 Stunden hydriert. Das nach Filtration und Waschen mit Tetrahydrofuran erhaltene Rohprodukt (25 g) enthielt 69% cis-Isomer und 25% trans-Isomer von 4-[trans-4-[2-(1,3-Dioxolan -2-yl)äthyl]cyclohexyl]cyclohexancarboxamid.

d) 42 g rohes 4-[trans-4-[2-(1,3-Dioxolan -2-yl)äthyl]cyclohexyl]cyclohexancarboxamid wurden unter Argonbegasung in 500 ml Aethylenglykol aufgeschlämmt und dann mit 19 g festem Kaliumhydroxid versetzt. Das Gemisch wurde unter Rühren 5 Stunden auf 180 °C (Badtemperatur) erhitzt. Nach dem Abkühlen wurde das Reaktionsgemisch auf 500 ml Wasser gegossen, mit 10-prozentiger Salzsäure auf pH ca. 3 angesäuert und dreimal mit je 300 ml Methylenchlorid extrahiert. Die vereinigten organischen Phasen wurden einmal mit 500 ml 1-prozentiger Salzsäure und zweimal mit je 500 ml Wasser gewaschen, über Magnesiumsulfat getrocknet, filtriert und eingedampft. Das dunkelbraune Rohprodukt (41 g) wurde an Kieselgel mit Aethylacetat chromatographisch gereinigt. Kristallisation des erhaltenen Produktes (36 g) aus Aceton ergab 13,8 g reine trans-4-[trans-4-[2-(1,3-Dioxolan -2-yl)äthyl]cyclohexyl]-cyclohexancarbonsäure. Aufarbeitung der Mutterlauge ergab weitere 2,5 g reines Produkt.

e) 18 g trans-4-[trans-4-[2-(1,3-Dioxolan-2-yl)äthyl]cyclohexyl]cyclohexancarbonsäure wurden unter Argonbegasung in 600 ml Chloroform gelöst und die Lösung bei 0 °C unter Rühren innert 3 Minuten tropfenweise mit einer Lösung von 7,2 ml Chlorameisensäureäthylester in 40 ml Chloroform versetzt. Die Reaktionslösung wurde noch 30 Minuten gerührt. Dann wurde während 10 Minuten Ammoniak-Gas in die Lösung eingeleitet. Das Gemisch wurde noch 30 Minuten bei 0 °C gerührt und dann zweimal mit je 300 ml Wasser extrahiert. Die wässrigen Phasen wurden mit je 100 ml Chloroform nachextrahiert. Die vereinigten organischen Phasen wurden über Magnesiumsulfat getrocknet, filtriert und eingedampft. Umkristallisation des erhaltenen, braunen kristallinen Rohproduktes (19 g) aus 800 ml Methylenchlorid ergab 13 g trans-4-[trans-4-[2-(1,3-Dioxolan -2-yl)äthyl]cyclohexyl]cyclohexancarboxamid als hellbraune Kristalle.

f) 2,1 g trans-4-[trans-4-[2-(1,3-Dioxolan -2-yl)äthyl]cyclohexyl]cyclohexancarboxamid wurden unter Argonbegasung in 60 ml Dimethylformamid aufgeschlämmt. Die Suspension wurde mit 1,32 ml Pyridin und 0,898 ml Methansulfochlorid versetzt und 1,5 Stunden bei 60 °C (Badtemperatur) gerührt. Anschliessend wurde die Reaktionslösung in Methylenchlorid und 10-prozentiger Salzsäure verteilt. Die wässrige Phase wurde zweimal mit je 100 ml Methylenchlorid extrahiert. Die organischen Phasen wurden zweimal mit je 100 ml Wasser gewaschen, über Magnesiumsulfat getrocknet, filtriert und eingeengt. Hierbei wurden 2,5 g trans-4-[trans-4-[2-(1,3-Dioxolan -2-yl)äthyl]cyclohexyl]cyclohexancarbonitril als gelbe Kristalle in einer Reinheit von 96% erhalten.

g) 3,0 g rohes trans-4-[trans-4-[2-(1,3-Dioxolan -2-yl)äthyl]cyclohexyl]cyclohexancarbonitril wurden unter Argonbegasung in 50 ml Wasser, 25 ml Eisessig und 10 ml Dioxan aufgeschlämmt und 1 Stnde bei 100 °C gerührt. Danach wurde die Reaktionslösung mit 100 ml Wasser versetzt. Die wässrige Phase wurde abgetrennt und dreimal mit je 100 ml Diäthyläther extrahiert. Die organischen Phasen wurden mit 100 ml verdünnter Natriumhydrogencarbonat-Lösung und mit 100 ml Wasser gewaschen, über Magnesiumsulfat getrocknet, filtriert und eingeengt. Umkristallisation der erhaltenen gelben Kristalle (2,25 g) aus

60 ml Hexan ergab 1,98 g 3-[trans-4-(trans-4-Cyanocyclohexyl)cyclohexyl]propionaldehyd als farblose Kristalle.

h) 4,11 g Methoxymethyl-triphenylphosphoniumchlorid wurden unter Argonbegasung in 60 ml tert.Butylmethyläther aufgeschlämmt und bei Raumtemperatur innert 2 Minuten mit 1,26 g Kalium-tert.butylat versetzt. Die Suspension wurde noch 1 Stunde bei Raumtemperatur gerührt, dann auf 0 °C gekühlt und innert 5 Minuten tropfenweise mit einer Lösung von 1,98 g 3-[trans-4-(trans-4-Cyanocyclohexyl)cyclohexyl]propionaldehyd in 25 ml tert.Butylmethyläther versetzt. Das Reaktionsgemisch wurde noch 45 Minuten bei 0 °C gerührt, dann mit 100 ml Wasser verdünnt und dreimal mit je 100 ml Diäthyläther extrahiert. Die organischen Phasen wurden zweimal mit je 100 ml Wasser gewaschen, über Magnesiumsulfat getrocknet, filtriert und eingeengt. Chromatographische Reinigung des Rohprodukts (5,1 g) an Kieselgel bei 0.5 bar mit Aethylacetat/Petroläther (Vol. 5:95) ergab 2,0 g trans-4-[trans-4-(4-Methoxy -3-butenyl)cyclohexyl]cyclohexancarbonitril als farbloses milchiges Oel.

i) 1,65 g trans-4-[trans-4-(4-Methoxy -3-butenyl)cyclohexyl]cyclohexancarbonitril wrden unter Argonbegasung in 50 ml Wasser, 25 ml Eisessig und 12 ml Dioxan aufgeschlämmt. Die Suspension wurde 2 Stunde: bei 80 °C (Badtemperatur) gerührt und dann mit 50 ml Wasser verdünnt. Die wässrige Phase wurde abgetrennt und dreimal mit je 100 ml Diäthyläther extrahiert. Die organischen Phasen wurden zweimal mit je 100 ml Wasser, dann mit 100 ml gesättigter Natriumhydrogencarbonat-Lösung und nochmals mit 100 ml Wasser gewaschen, dann über Magnesiumsulfat getrocknet, filtriert und eingeengt. Hierbei wurden 1,5 g 4-[trans-4-(trans-4-Cyanocyclohexyl)cyclohexyl]butyraldehyd als farblose Kristalle erhalten.

j) 3,28 g Methyltriphenylphosphoniumbromid wurden unter Argonbegasung in 40 ml tert.Butylmethyläther aufgeschlämmt. Die Suspension wurde bei Raumtemperatur innert 1 Minute mit 962 mg Kalium-tert.butylat versetzt und 1 Stunde gerührt. Anschliessend wurde das Gemisch auf 0 °C gekühlt, innert 3 Minuten tropfenweise mit einer Lösung von 1,5 g 4-[trans-4-(trans-4-Cyanocyclohexyl)cyclohexyl]-butyraldehyd in 20 ml tert.Butylmethyläther versetzt und noch 45 Minuten bei 0 °C gerührt. Danach wurde das Reaktionsgemisch mit 80 ml Wasser verdünnt und dreimal mit je 100 ml Petroläther extrahiert. Die organichen Phasen wurden zweimal mit je 100 ml Wasser gewaschen, über Magnesiumsulfat getrocknet, filtriert und eingeengt. Chromtographische Reinigung des Rohproduktes (2.1 g) an Kieselgel bei 0,5 bar mit Aethylacetat/Petroläther (Vol. 2:98) und Umkristallisation aus 20 ml Methanol ergab 1,26 g trans-4-[trans-4-(4-Pentenyl)cyclohexyl]cyclohexancarbonitril als farblose Kristalle mit Smp. (C-$S_B$) 20,1 °C, $S_B$-N 36,9 °C, Klp. (N-I) 54,8 °C.

In analoger Weise können folgende Verbindungen hergestellt werden:

trans-4-[trans-4-(4Z-Hexenyl)cyclohexyl]cyclohexancarbonitril;
trans-4-[trans-4-(4Z-Heptenyl)cyclohexyl]cyclohexancarbonitril;
trans-4-[trans-4-(4Z-Octenyl)cyclohexyl]cyclohexancarbonitril;
trans-4-[trans-4-(4Z-Nonenyl)cyclohexyl]cyclohexancarbonitril;
trans-4-[trans-4-(4Z-Decenyl)cyclohexyl]cyclohexancarbonitril.

Durch Umsetzung des in Stufe g) erhaltenen 3-[trans-4-(trans-4-Cyanocyclohexyl)cyclohexyl]-propionaldehyds in analoger Weise zu Stufe j) und gegebenenfalls E/Z-Isomerisierung in analoger Weise zu Beispiel 1k) können folgende Verbindungen hergestellt werden:

trans-4-[trans-4-(3-Butenyl)cyclohexyl]cyclohexancarbonitril; Smp. (C-N) 50,7 °C, Klp. (N-I) 82,7 °C;
trans-4-[trans-4-(3E-Pentenyl)cyclohexyl]cyclohexancarbonitril; Smp. (C-N) 79,4 °C, (N-I) 99,5 °C;
trans-4-[trans-4-(3E-Hexenyl)cyclohexyl]cyclohexancarbonitril;
trans-4-[trans-4-(3E-Heptenyl)cyclohexyl]cyclohexancarbonitril;
trans-4-[trans-4-(3E-Octenyl)cyclohexyl]cyclohexancarbonitril;
trans-4-[trans-4-(3E-Nonenyl)cyclohexyl]cyclohexancarbonitril;
trans-4-[trans-4-(3E-Decenyl)cyclohexyl]cyclohexancarbonitril.

Durch Umsetzung des 4-(4-Oxocyclohexyl)benzamids in analoger Weise zu den Stufen h) und i), Acetalisierung des durch Kristallisation erhaltenen 4-(trans-4-Formylcyclohexyl)benzamids mit Aethylenglykol in Gegenwart von p-Toluolsulfonsäure, weitere Umsetzung des Dioxolans in analoger Weise zu den Stufen c) bis g), anschliessende Wittig-Reaktion in analoger Weise zu Stufe j) und gegebenenfalls E/Z-Isomerisierung in analoger Weise zu Beispiel 1k) können folgende Verbindungen hergestellt werden:

trans-4-(trans-4-Vinylcyclohexyl)cyclohexancarbonitril, Smp. (C-N) 55,7 °C, Klp. (N-I) 59 °C;
trans-4-[trans-4-(1E-Propenyl)cyclohexyl]cyclohexancarbonitril, Smp. (C-N) 64,9 °C, Klp. (N-I) 100,1 °C;
trans-4-[trans-4-(1E-Butenyl)cyclohexyl]cyclohexancarbonitril, Smp. (C-S) 38,5 °C, Phasenübergang (S-N) 59 °C, Klp. (N-I) 91 °C;
trans-4-[trans-4-(1E-Pentenyl)cyclohexyl]cyclohexancarbonitril, Smp. (C-N) 59,3 °C, Klp. (N-I) 91,7 °C;
trans-4-[trans-4-(1E-Hexenyl)cyclohexyl]cyclohexancarbonitril;

trans-4-[trans-4-(1E-Heptenyl)cyclohexyl]cyclohexancarbonitril;
trans-4-[trans-4-(1E-Octenyl)cyclohexyl]cyclohexancarbonitril;
trans-4-[trans-4-(1E-Noneyl)cyclohexyl]cyclohexancarbonitril;
trans-4-[trans-4-(1E-Decenyl)cyclohexyl]cyclohexancarbonitril.

Ausgehend von 4-[2-(4-Oxocyclohexyl)äthyl]benzamid [herstellbar aus dem in Mol. Cryst. Liq. Cryst. 131, 327 (1985) beschriebenen Nitril] können in analoger Weise zur obigen Methode auch die folgenden Verbindungen hergestellt werden:

trans-4-[2-(trans-4-(4-Pentenyl)cyclohexyl)äthyl]cyclohexancarbonitril;
trans-4-[2-(trans-4-(4Z-Hexenyl)cyclohexyl)äthyl]cyclohexancarbonitril;
trans-4-[2-(trans-4-(4Z-Heptenyl)cyclohexyl)äthyl]cyclohexancarbonitril;
trans-4-[2-(trans-4-(4Z-Octenyl)cyclohexyl)äthyl]cyclohexancarbonitril;
trans-4-[2-(trans-4-(4Z-Nonenyl)cyclohexyl)äthyl]cyclohexancarbonitril;
trans-4-[2-(trans-4-(4Z-Decenyl)cyclohexyl)äthyl]cyclohexancarbonitril;
trans-4-[2-(trans-4-(3-Butenyl)cyclohexyl)äthyl]cyclohexancarbonitril;
trans-4-[2-(trans-4-(3E-Pentenyl)cyclohexyl)äthyl]cyclohexancarbonitril;
trans-4-[2-(trans-4-(3E-Hexenyl)cyclohexyl)äthyl]cyclohexancarbonitril;
trans-4-[2-(trans-4-(3E-Heptenyl)cyclohexyl)äthyl]cyclohexancarbonitril;
trans-4-[2-(trans-4-(3E-Octenyl)cyclohexyl)äthyl]cyclohexancarbonitril;
trans-4-[2-(trans-4-(3E-Nonenyl)cyclohexyl)äthyl]cyclohexancarbonitril;
trans-4-[2-(trans-4-(3E-Decenyl)cyclohexyl)äthyl]cyclohexancarbonitril;
trans-4-[2-(trans-4-Vinylcyclohexyl)äthyl]cyclohexancarbonitril;
trans-4-[2-(trans-4-(1E-Propenyl)cyclohexyl)äthyl]cyclohexancarbonitril;
trans-4-[2-(trans-4-(1E-Butenyl)cyclohexyl)äthyl]cyclohexancarbonitril;
trans-4-[2-(trans-4-(1E-Pentenyl)cyclohexyl)äthyl]cyclohexancarbonitril;
trans-4-[2-(trans-4-(1E-Hexenyl)cyclohexyl)äthyl]cyclohexancarbonitril;
trans-4-[2-(trans-4-(1E-Heptenyl)cyclohexyl)äthyl]cyclohexancarbonitril;
trans-4-[2-(trans-4-(1E-Octenyl)cyclohexyl)äthyl]cyclohexancarbonitril;
trans-4-[2-(trans-4-(1E-Nonenyl)cyclohexyl)äthyl]cyclohexancarbonitril;
trans-4-[2-(trans-4-(1E-Decenyl)cyclohexyl)äthyl]cyclohexancarbonitril.

## Beispiel 3

a) Eine Lösung von 1,71 g trans-4-[trans-4-(4-Pentenyl) cyclohexyl]cyclohexancarbonitril (hergestellt nach Beispiel 2) in 30 ml Diäthylenglykol wurde mit 3,11 g Kaliumhydroxid versetzt und 3 Stunden bei 130 °C gerührt. Dann wurde das Gemisch auf Eiswasser gegossen, mit 25-prozentiger Salzsäure angesäuert und dreimal mit Diäthyläther extrahiert. Die organischen Phasen wurden vereinigt, dreimal mit Wasser gewaschen, über Magnesiumsulfat getrocknet und eingedampft. Umkristallisation des braunen, kristallinen Rückstandes (1.74 g) aus 30 ml Hexan ergab 996 mg trans-4-[trans-4-(4-Pentenyl)-cyclohexyl]cyclohexancarbonsäure als gelbliche Kristalle.

b) Eine Lösung von 996 mg trans-4-[trans-4-(4-Pentenyl)cyclohexyl]cyclohexancarbonsäure in 80 ml Methylenchlorid wurde mit 752 mg p-Fluorphenol, 61 mg 4-(Dimethylamino)pyridin und 1,03 g Dicyclohexylcarbodiimid versetzt und 15 Stunden bei Raumtemperatur gerührt. Anschliessend wurde das Reaktionsgemisch filtriert. Das Filtrat wurde eingedampft und der erhaltene Rückstand an Kieselgel mit Aethylacetat/Petroläther (Vol. 3:97) chromatographisch gereinigt. Kristallisation des erhaltenen Produktes (1,24 g) aus 40 ml Hexan ergab 757 mg reinen trans-4-[trans-4-(4-Pentenyl)cyclohexyl]-cyclohexancarbonsäure -4-fluorphenylester mit Smp. (C-N) 70,3 °C und Klp. (N-I) 158,7 °C.

In analoger Weise können folgende Verbindungen hergestellt werden:

trans-4-(4-Pentenyl)cyclohexancarbonsäure-4-fluorphenylester, Smp. 31.3 °C;
trans-4-(4-Pentenyl)cyclohexancarbonsäure-3,4-difluorphenylester, Smp. 12,4 °C;
trans-4-(4-Pentenyl)cyclohexancarbonsäure-4-chlorphenylester;
4-(3-Butenyloxy)benzoesäure-4-fluorphenylester, Smp. 65 °C;
4-(3-Butenyloxy)benzoesäure-3,4-difluorphenylester, Smp. 47,5 °C;
4-(3-Butenyloxy)benzoesäure-4-chlorphenylester;
trans-4-[trans-4-(4-Pentenyl)cyclohexyl]cyclohexancarbonsäure-3,4-difluorphenylester;
trans-4-[trans-4-(4-Pentenyl)cyclohexyl]cyclohexancarbonsäure-4-chlorphenylester;
trans-4-[trans-4-(4-Pentenyl)cyclohexyl]cyclohexancarbonsäure-3-chlor-4-fluorphenylester;
trans-4-[trans-4-(4-Pentenyl)cyclohexyl]cyclohexancarbonsäure-3-fluor-4-chlorphenylester;
4-[trans-4-(4-Pentenyl)cyclohexyl]benzoesäure-4-fluorphenylester;

4-[trans-4-(4-Pentenyl)cyclohexyl]benzoesäure-3,4-difluorphenylester;
5-[trans-4-(4-Pentenyl)cyclohexyl]-2-pyrimidincarbonsäure-4-fluorphenylester;
2-[trans-4-(4-Pentenyl)cyclohexyl]-5-pyrimidincarbonsäure4-fluorphenylester;
4-[5-(4-Pentenyl)-2-pyrimidinyl]benzoesäure-4-fluorphenylester;
4-[2-(4-Pentenyl)-5-pyrimidinyl]benzoesäure-4-fluorphenylester;
trans-4-(4Z-Hexenyl)cyclohexancarbonsäure-4-fluorphenylester;
trans-4-(4Z-Hexenyl)cyclohexancarbonsäure-3,4-difluorphenylester;
4-(3Z-Pentenyloxy)benzoesäure-4-fluorphenylester;
trans-4-[trans-4-(4Z-Hexenyl)cyclohexyl]cyclohexancarbonsäure-4-fluorphenylester;
4-[trans-4-(4Z-Hexenyl)cyclohexyl]benzoesäure-4-fluorphenylester;
trans-4-(3-Butenyl)cyclohexancarbonsäure-3,4-difluorphenylester, Smp. 42,0 °C;
trans-4-(3-Butenyl)cyclohexancarbonsäure-4-chlorphenylester;
4-Allyloxybenzoesäure-4-fluorphenylester;
4-Allyloxybenzoesäure-3,4-difluorphenylester;
4-Allyloxybenzoesäure-4-chlorphenylester;
trans-4-[trans-4-(3-Butenyl)cyclohexyl]cyclohexancarbonsäure-3,4-difluorphenylester, Smp. (C-N) 55,1 °C, Klp. (N-I) 153,6 °C;
trans-4-[trans-4-(3-Butenyl)cyclohexyl]cyclohexancarbonsäure-4-chlorphenylester, Smp. (C-N) 78,3 °C, Klp. (N-I) 213,5 °C;
trans-4-[trans-4-(3-Butenyl)cyclohexyl]cyclohexancarbonsäure-3-chlor-4-fluorphenylester;
trans-4-[trans-4-(3-Butenyl)cyclohexyl]cyclohexancarbonsäure-3-fluor-4-chlorphenylester;
4-[trans-4-(3-Butenyl)cyclohexyl]benzoesäure-3,4-difluorphenylester;
trans-4-(3E-Pentenyl)cyclohexancarbonsäure-3,4-difluorphenylester;
4-(2E-Butenyloxy)benzoesäure-4-fluorphenylester.

Beispiel 4

a) Eine Grignard Lösung aus 3,59 g Magnesium und 16,75 ml 1-Brom-4 fluorbenzol in 70 ml Tetrahydrofuran wurde bei 0 °C innert 30 Minuten tropfenweise mit einer Lösung von 33,1 g 4-[trans-4 -[2-(1,3-Dioxolan-2-yl)äthyl]cyclohexyl]cyclohexanon (hergestellt nach Beispiel 1) in 90 ml Tetrahydrofuran versetzt. Das Reaktionsgemisch wurde noch 4 Stunden bei Raumtemperatur gerührt und dann 1,5 Stunden zum Sieden erhitzt. Anschliessend wurde das Reaktionsgemisch abkühlen gelassen, mit 100 ml Diäthyläther verdünnt und mit 80 ml halbgesättigter Ammoniumchlorid-Lösung gewaschen. Die wässrige Phase wurde mit 100 ml Diäthyläther nachextrahiert. Die vereinigten organischen Phasen wurden dreimal mit je 60 ml gesättigter Kochsalzlösung gewaschen, über Natriumsulfat getrocknet, filtriert und eingedampft. Hierbei wurden 41,0 g rohes 1-[4-[trans-4-(2-(1,3-Dioxolan-2-yl)äthyl)cyclohexyl] -1-hydroxycyclohexyl]-4-fluorbenzol erhalten.

b) Eine Lösung von 41,0 g rohem 1-[4-[trans-4-(2-(1,3-Dioxolan-2-yl)äthyl]cyclohexyl] -1-hydroxycyclohexyl]-4-fluorbenzol in 225 ml m-Xylol wurde mit 15 g Kaliumhydrogensulfat versetzt und das Gemisch unter Rühren 11 Stunden zum Sieden erhitzt. Nach dem Erkalten wurde das Salz abfiltriert. Das Filtrat wurde mit 250 ml Diäthyläther verdünnt, mit 200 ml gesättigter Natriumhydrogencarbonat-Lösung und zweimal mit je 150 ml Wasser gewaschen, über Natriumsulfat getrocknet und eingeengt. Hierbei wurden 30,3 g rohes 1-[4-[trans-4-(2-(1,3-Dioxolan-2-yl)äthyl)cyclohexyl] -1-cyclohexenyl]-4-fluorbenzol erhalten.

c) Eine Lösung von 31,5 g rohem 1-[4-[trans-4-(2-(1,3-Dioxolan-2-yl)äthyl)cyclohexyl] -1-cyclohexenyl]-4-fluorbenzol und 0,5 ml Triäthylamin in 1 l Toluol wurde mit 4,5 g 5-prozentiger Palladium-Kohle bei Raumtemperatur und Normaldruck hydriert, bis die Wasserstoffaufnahme zum Stillstand kam. Der Katalysator wurde abfiltriert und das Filtrat eingedampft. Das erhaltene 1-[4-[trans-4-(2-(1,3-Dioxolan -2-yl)äthyl)cyclohexyl]cyclohexyl]-4-fluorbenzol (31,1 g; cis/trans-Verhältnis ca. 1:1) wurde zur Isomerisierung mit einer Lösung von 10,0 g Kalium-tert.-butylat in 310 ml N,N-Dimethylformamid versetzt und 23 Stunden auf 105 °C erhitzt. Anschliessend wurde das Reaktionsgemisch auf 400 g Eis und 100 ml gesättigte Natriumhydrogencarbonat-Lösung gegossen. Das Gemisch wurde einmal mit 500 ml und zweimal mit je 250 ml Diäthyläther extrahiert. Die organischen Phasen wurden dreimal mit je 200 ml Wasser gewaschen, über Natriumsulfat getrocknet, filtriert und eingedampft. Hierbei wurden 30,5 g überwiegend festes 1-[trans-4-[trans-4-(2-(1,3-Dioxolan -2-yl)äthyl)cyclohexyl]cyclohexyl]-4-fluorbenzol erhalten, welches ohne zusätzliche Reinigung weiterverwendet wurde.

In analoger Weise können folgende Verbindungen hergestellt werden:
1-[trans-4-[trans-4-(2-(1,3-Dioxolan-2-yl)äthyl)cyclohexyl]cyclohexyl]-3,4-difluorbenzol;

1-[trans-4-[trans-4-(2-(1,3-Dioxolan-2-yl)äthyl)cyclohexyl]cyclohexyl]-3-chlor-4-fluorbenzol;
1-[trans-4-[trans-4-(2-(1,3-Dioxolan-2-yl)äthyl)cyclohexyl]cyclohexyl]-3-fluor-4-chlorbenzol;
1-[trans-4-[trans-4-(2-(1,3-Dioxolan-2-yl)äthyl)cyclohexyl]cyclohexyl]-4-chlorbenzol;
1-[trans-4-[2-[trans-4-(2-(1,3-Dioxolan-2-yl)äthyl)cyclohexyl]äthyl]cyclohexyl]-4-fluorbenzol;
1-[trans-4-[2-[trans-4-(2-(1,3-Dioxolan-2-yl)äthyl)cyclohexyl]äthyl]cyclohexyl]-3,4-difluorbenzol;
1-[trans-4-[2-[trans-4-(2-(1,3-Dioxolan-2-yl)äthyl)cyclohexyl]äthyl]cyclohexyl]-3-chlor-4-fluorbenzol;
1-[trans-4-[2-[trans-4-(2-(1,3-Dioxolan-2-yl)äthyl)cyclohexyl]äthyl]cyclohexyl]-4-chlorbenzol;
1-[trans-4-[2-[trans-4-(2-(1,3-Dioxolan-2 yl)äthyl)cyclohexyl]äthyl]cyclohexyl]-3-fluor-4-chlorbenzol.

Beispiel 5

a) Eine Lösung von 3,1 g trans-4-[trans-4 -[2-(1,3-Dioxolan-2-yl)äthyl]cyclohexyl]cyclohexancarbonsäure (hergestellt nach Beispiel 2) in 50 ml trockenem Diäthyläther wird bei 0 °C mit 380 mg Lithiumaluminiumhydrid versetzt und dann 4 Stunden zum Rückfluss erhitzt. Danach wird das Reaktionsgemisch abgekühlt, mit Eiswasser und Ammoniumchlorid-Lösung versetzt und mit Diäthyläther extrahiert. Die organische Phase wird mit Wasser gewaschen, über Magnesiumsulfat getrocknet, filtriert und eingedampft. Das erhaltene Rohprodukt von [trans-4-[trans-4 -(2-(1,3-Dioxolan-2-yl)äthyl)cyclohexyl]-cyclohexyl]methanol wird ohne zusätzliche Reinigung weiterverwendet.

b) Eine Lösung von 2,96 g rohem [trans-4-[trans-4 -(2-(1,3-Dioxolan-2-yl)äthyl)cyclohexyl]cyclohexyl]-methanol in 20 ml trockenem Pyridin wird bei 0 °C mit 2,1 g p-Toluolsulfonylchlorid versetzt. Das Gemisch wird 15 Stunden bei Raumtemperatur gerührt, dann mit 200 ml Methylenchlorid verdünnt und mehrmals mit Wasser gewaschen. Die organische Phase wird über Magnesiumsulfat getrocknet, filtriert und eingedampft. Chromatographische Reinigung des erhaltenen Rohproduktes an Kieselgel mit Aethylacetat/Petroläther (Vol. 1:9) ergibt [trans-4-[trans-4 -(2-(1,3-Dioxolan-2-yl)äthyl)cyclohexyl]-cyclohexyl]methyl -p-toluolsulfonat.

c) Eine Lösung von 0,33 g Kaliumhydroxid in 7 ml 95-prozentigem Aethanol wird mit 1,12 g p-Fluorphenol versetzt. Danach wird das Gemisch mit einer Lösung von 2,26 g [trans-4-[trans-4 -(2-(1,3-Dioxolan-2-yl)äthyl)cyclohexyl]cyclohexyl]methyl -p-toluolsulfonat in 23 ml Aethanol versetzt und 24 Stunden bei 80 °C (Badtemperatur) gerührt. Anschliessend wird das Reaktionsgemisch in 1N Salzsäure und Methylenchlorid verteilt. Die organische Phase wird mehrmals mit Wasser gewaschen, über Magnesiumsulfat getrocknet, filtriert und eingedampft. Chromatographische Reinigung des erhaltenen Rohproduktes an Kieselgel mit Aethylacetat/Petroläther ergibt 1-[[trans-4-(trans-4- (2-(1,3-Dioxolan-2-yl)-äthyl)cyclohexyl)cyclohexyl]methoxy] -4-fluorbenzol.

In analoger Weise können folgende Verbindungen hergestellt werden:
1-[[trans-4-(trans-4-(2-(1,3-Dioxolan-2-yl)-äthyl)cyclohexyl)cyclohexyl]methoxy]-3,4-difluorbenzol;
1-[[trans-4-(trans-4-(2-(1,3-Dioxolan-2-yl)-äthyl)cyclohexyl)cyclohexyl]methoxy]-3-chlor-4-fluorbenzol;
1-[[trans-4-(trans-4-(2-(1,3-Dioxolan-2-yl)-äthyl)cyclohexyl)cyclohexyl]methoxy]-4-chlorbenzol;
1-[[trans-4-(trans-4-(2-(1,3-Dioxolan-2-yl)-äthyl)cyclohexyl)cyclohexyl]methoxy]-3-fluor-4-chlorbenzol.

Beispiel 6

a) Eine Lösung von 3,1 g Pyridiniumchlorochromat in 20 ml Methylenchlorid wird bei Raumtemperatur tropfenweise mit einer Lösung von 3 g [trans-4-[trans-4 -(2-(1,3-Dioxolan-2-yl)äthyl)cyclohexyl]-cyclohexyl]methanol (herstellbar nach Beispiel 5) in 10 ml Methylenchlorid versetzt. Das Gemisch wird noch 1 Stunde gerührt, dann mit 50 ml Diäthyläther verdünnt und filtriert. Das Filtrat wird eingedampft, der Eindampfrückstand in 50 ml Diäthyläther aufgenommen und die erhaltene Lösung nochmals filtriert. Chromatographische Reinigung an Kieselgel mit Aethylacetat/Petroläther (Vol. 1:4) und Kristallisation aus Aethylacetat/Hexan ergibt schliesslich trans-4-[trans-4-[2-(1,3-Dioxolan -2-yl)äthyl]cyclohexyl]-cyclohexancarboxaldehyd.

b) 3 g p-Fluorbenzyl-triphenylphosphoniumbromid (herstellbar aus p-Fluorbenzylbromid und Triphenylphosphin) werden in 50 ml tert.Butylmethyläther aufgeschlämmt. Die Suspension wird bei Raumtemperatur mit 0,75 g Kalium-tert.butylat versetzt und 1,5 Stunden gerührt. Anschliessend wird das Gemisch bei 0 °C innert 5 Minuten tropfenweise mit einer Lösung von 1,40 g trans-4-[trans-4-[2-(1,3-Dioxoalan -2-yl)-äthyl]cyclohexyl]cyclohexancarboxaldehyd in 25 ml tert.Butylmethyläther versetzt und noch 24 Stunden bei Raumtemperatur gerührt. Danach wird das Reaktionsgemisch in Diäthyläther aufgenommen, mehrmals mit Wasser gewaschen, über Magnesiumsulfat getrocknet, filtriert und eingeengt. Chromatographische Reinigung des erhaltenen Rohproduktes an Kieselgel mit Aethylacetat/Petroläther (Vol. 3:97) ergibt ß-[trans-4-[trans-4-(2-(1,3-Dioxolan -2-yl)äthyl)cyclohexyl]cyclohexyl]-4-fluorstyrol.

c) Eine Lösung von 1 g ß-[trans-4-[trans-4-(2-(1,3-Dioxolan -2-yl)äthyl)cyclohexyl]cyclohexyl]-4-fluorstyrol in 10 ml Toluol und 5 ml Aethanol wird mit 500 mg 5-prozentiger Palladium-Kohle bei Raumtemperatur und Normaldruck bis zum Stillstand der Wasserstoffaufnahme hydriert. Anschliessend wird die schwarze Suspension filtriert. Eindampfen des Filtrates ergibt 1-[2-[trans-4-[trans-4-(2-(1,3-Dioxolan -2-yl)äthyl)-cyclohexyl]cyclohexyl]äthyl]-4-fluorbenzol.

In analoger Weise können folgende Verbindungen hergestellt werden:
1-[2-[trans-4-[trans-4-(2-(1,3-Dioxolan-2-yl)äthyl)cyclohexyl]cyclohexyl]äthyl]-3,4-difluorbenzol;
1-[2-[trans-4-[trans-4-(2-(1,3-Dioxolan-2-yl)äthyl)cyclohexyl]cyclohexyl]äthyl]-3-chlor-4-fluorbenzol;
1-[2-[trans-4-[trans-4-(2-(1,3-Dioxolan-2-yl)äthyl)cyclohexyl]cyclohexyl]äthyl]-4-chlorbenzol;
1-[2-[trans-4-[trans-4-(2-(1,3-Dioxolan-2-yl)äthyl)cyclohexyl]cyclohexyl]äthyl]-3-fluor-4-chlorbenzol.

Beispiel 7

Eine Lösung von 1,7 g 4-Fluorbenzoylchlorid in 5 ml Pyridin wird mit 2,8 g trans-4-[trans -4-[2-(1,3-Dioxolan-2-yl)äthyl]cyclohexyl]cyclohexanol (hergestellt nach Beispiel 1) versetzt und 12 Stunden bei Raumtemperatur gerührt. Danach wird das Reaktionsgemisch auf Eiswasser gegossen und dreimal mit Diäthyläther extrahiert. Die organischen Phasen werden nacheinander mit gesättigter Natriumhydrogencarbonat-Lösung, mit 10-prozentiger Salzsäure, mit gesättigter Natriumhydrogencarbonat-Lösung und mit Wasser gewaschen, dann über Magnesiumsulfat getrocknet, filtriert und eingeengt. Chromatographische Reinigung des erhaltenen Rohproduktes an Kieselgel mit Aethylacetat/Petroläther (Vol. 3:97) ergibt 4 Fluorbenzoesäure-trans-4-[trans-4-[2-(1,3-dioxolan -2-yl)äthyl]cyclohexyl]cyclohexylester.

In analoger Weise können folgende Verbindungen hergestellt werden:
3,4-Difluorbenzoesäure-trans-4-[trans-4-[2-(1,3-dioxolan-2-yl)äthyl]cyclohexyl]cyclohexylester;
3-Chlor-4-fluorbenzoesäure-trans-4-[trans-4-[2-(1,3-dioxolan-2-yl)äthyl]cyclohexyl]cyclohexylester;
4-Chlorbenzoesäure-trans-4-[trans-4-[2-(1,3-dioxolan-2-yl)äthyl]cyclohexyl]cyclohexylester;
3-Fluor-4-chlorbenzoesäure-trans-4-[trans-4-[2-(1,3-dioxolan-2-yl)äthyl]cyclohexyl]cyclohexylester.

Beispiel 8

2,04 g Natriumhydrid als ca. 50-prozentige ölige Suspension werden unter Stickstoffbegasung vorgelegt und zweimal mit Pentan gewaschen. Dann werden zum Natriumhydrid 40 ml trockenes Tetrahydrofuran und eine Lösung von 6.0 g trans-4-[trans -4-[2-(1,3-Dioxolan-2-yl)äthyl]cyclohexyl]cyclohexanol (hergestellt nach Beispiel 2) in 30 ml Tetrahydrofuran zugefügt. Das Gemisch wird bei Raumtemperatur 30 Minuten gerührt, dann mit 6,03 g 4-Fluorbenzylbromid versetzt und 2 Stunden zum Rückfluss erhitzt. Anschliessend wird das Reaktionsgemisch abgekühlt, in 200 ml Diäthyläther aufgenommen und dreimal mit je 200 ml Wasser gewaschen. Die organische Phase wird über Magnesiumsulfat getrocknet, filtriert und eingedampft, wobei trans-4-[trans-4-[2-(1,3-Dioxolan -2-yl)äthyl]cyclohexyl]cyclohexyl-4-fluorbenzyläther erhalten wird.

In analoger Weise können folgende Verbindungen hergestellt werden:
trans-4-[trans-4-[2-(1,3-Dioxolan-2-yl)äthyl]cyclohexyl]cyclohexyl-3,4-difluorbenzyläther;
trans-4-[trans-4-[2-(1,3-Dioxolan-2-yl)äthyl]cyclohexyl]cyclohexyl-3-chlor-4-fluorbenzyläther;
trans-4-[trans-4-[2-(1,3-Dioxolan-2-yl)äthyl]cyclohexyl]cyclohexyl-4-chlorbenzyläther;
trans-4-[trans-4-[2-(1,3-Dioxolan-2-yl)äthyl]cyclohexyl]cyclohexyl-3-fluor-4-chlorbenzyläther.

Beispiel 9

a) Ein Gemisch von 29.1 g rohem 1-[trans-4-[trans-4-(2-(1,3-Dioxolan -2-yl)äthyl)cyclohexyl]cyclohexyl]-4-fluorbenzol, 200 ml Dioxan, 200 ml Eisessig und 400 ml Wasser wurde unter Rühren und Stickstoffbegasung 5 Stunden zu leichtem Sieden erhitzt (Badtemperatur 115 °C). Dann wurde das Reaktionsgemisch auf 500 g Eis gegossen. Die wässrige Phase wurde abgetrennt und dreimal mit je 400 ml Diäthyläther extrahiert. Die vereinigten organischen Phasen wurden mit 500 ml gesättigter Natriumhydrogencarbonat-Lösung und mit 500 ml Wasser gewaschen, über Natriumsulfat getrocknet, filtriert und eingeengt. Hierbei wurden 25,2 g roher, fester 3-[trans-4-[trans -4-(4-Fluorphenyl)cyclohexyl]-cyclohexyl]propionaldehyd erhalten.

b) Eine Suspension von 9,1 g Methoxymethyl-triphenylphosphoniumchlorid in 50 ml Diäthyläther wurde unter Stickstoffbegasung mit 2,85 g Kalium-tert.butylat versetzt. Die rote Suspension wurde noch 30 Minuten bei Raumtemperatur gerührt und dann bei 0 °C tropfenweise mit einer Lösung von 5,43 g rohem 3-[trans-4 -[trans-4-(4-Fluorphenyl)cyclohexyl]cyclohexyl]propionaldehyd in 30 ml trockenem Diäthyläther versetzt. Das Reaktionsgemisch wurde noch 90 Minuten bei Raumtemperatur gerührt, dann auf 300 ml

Hexan gegossen und filtriert. Chromatogrpahische Reinigung des eingeengten Filtrates an Kieselgel mit Hexan ergab 4,9 g festes 1-[trans-4-[trans-4-(4-Methoxy -3-butenyl)cyclohexyl]cyclohexyl]-4-fluorbenzol.

c) Unter Rühren und Stickstoffbegasung wurde ein Gemisch von 2,48 g 1-[trans-4-[trans-4-(4-Methoxy -3-butenyl)cyclohexyl]cyclohexyl]-4-fluorbenzol, 30 ml Dioxan, 20 ml Eisessig und 40 ml Wasser 6 Stunden zu leichtem Sieden erhitzt (Badtemperatur 115 °C). Nach dem Erkalten wurde die Suspension mit 70 ml Wasser verdünnt. Die wässrige Phase wurde abgetrennt und dreimal mit je 80 ml Diäthyläther extrahiert. Die vereinigten organischen Phasen wurden zweimal mit je 100 ml Wasser gewaschen, über Natriumsulfat getrocknet, filtriert und eingeengt. Hierbei wurden 2,3 g fester 4-[trans-4-[trans -4-(4-Fluorphenyl)cyclohexyl]cyclohexyl]butyraldehyd erhalten.

d) Eine Suspension von 5,19 g Methyl-triphenylphosphoniumbromid in 80 ml Diäthyläther wurde unter Stickstoffbegasung mit 1,55 g Kalium-tert.butylat versetzt. Die gelbe Suspension wurde noch 45 Minuten bei Raumtemperatur gerührt und dann bei 0 °C tropfenweise mit einer Lösung von 2,3 g 4-[trans-4-[trans -4-(4-Fluorphenyl)cyclohexyl]cyclohexyl]butyraldehyd versetzt. Das Reaktionsgemisch wurde noch 2 Stunden bei 0 °C gerührt und dann mit 60 ml Wasser verdünnt. Die wässrige Phase wurde abgetrennt und zweimal mit je 60 ml Hexan extrahiert. Die vereinigten organischen Phasen wurden mit Wasser neutral gewaschen, über Natriumsulfat getrocknet, filtriert und eingeengt. Chromatographische Reinigung des Rückstandes an Kieselgel mit Hexan ergab 1,89 g Rohprodukt. Nach zweimaliger Umkristallisation aus Aceton bei -20 °C wurden 1,22 g 1-[trans-4-[trans-4-(4-Pentenyl)cyclohexyl]cyclohexyl] -4-fluorbenzol mit Smp. (C-N) 65,7 °C und Klp. (N-I) 129,7 °C erhalten.

In analoger Weise können die in Beispiel 3 genannten 4-Alkenylverbindungen sowie die folgenden Verbindungen hergestellt werden:

1-[trans-4-[trans-4-(4-Pentenyl)cyclohexyl]cyclohexyl-3,4-difluorbenzol;
1-[trans-4-[trans-4-(4-Pentenyl)cyclohexyl]cyclohexyl-3-chlor-4-fluorbenzol;
1-[trans-4-[trans-4-(4-Pentenyl)cyclohexyl]cyclohexyl-4-chlorbenzol;
1-[trans-4-[trans-4-(4-Pentenyl)cyclohexyl]cyclohexyl-3-fluor-4-chlorbenzol;
1-[trans-4-[2-(trans-4-(4-Pentenyl)cyclohexyl)äthyl]cyclohexyl]-4-fluorbenzol;
1-[trans-4-[2-(trans-4-(4-Pentenyl)cyclohexyl)äthyl]cyclohexyl]-3,4-difluorbenzol;
1-[trans-4-[2-(trans-4-(4-Pentenyl)cyclohexyl)äthyl]cyclohexyl]-3-chlor-4-fluorbenzol;
1-[trans-4-[2-(trans-4-(4-Pentenyl)cyclohexyl)äthyl]cyclohexyl]-4-chlorbenzol;
1-[trans-4-[2-(trans-4-(4-Pentenyl)cyclohexyl)äthyl]cyclohexyl]-3-fluor-4-chlorbenzol;
1-[[trans-4-(trans-4-(4-Pentenyl)cyclohexyl)cyclohexyl]methoxy]-4-fluorbenzol;
1-[[trans-4-(trans-4-(4-Pentenyl)cyclohexyl)cyclohexyl]methoxy]-3,4-difluorbenzol;
1-[[trans-4-(trans-4-(4-Pentenyl)cyclohexyl)cyclohexyl]methoxy]-3-chlor-4-fluorbenzol;
1-[[trans-4-(trans-4-(4-Pentenyl)cyclohexyl)cyclohexyl]methoxy]-4-chlorbenzol;
1-[[trans-4-(trans-4-(4-Pentenyl)cyclohexyl)cyclohexyl]methoxy]-3-fluor-4-chlorbenzol;
1-[2-[trans-4-(trans-4-(4-Pentenyl)cyclohexyl)cyclohexyl]äthyl]-4-fluorbenzol;
1-[2-[trans-4-(trans-4-(4-Pentenyl)cyclohexyl)cyclohexyl]äthyl]-3,4-difluorbenzol;
1-[2-[trans-4-(trans-4-(4-Pentenyl)cyclohexyl)cyclohexyl]äthyl]-3-chlor-4-fluorbenzol;
1-[2-[trans-4-(trans-4-(4-Pentenyl)cyclohexyl)cyclohexyl]äthyl]-4-chlorbenzol;
1-[2-[trans-4-(trans-4-(4-Pentenyl)cyclohexyl)cyclohexyl]äthyl]-3-fluor-4-chlorbenzol;
4-Fluorbenzoesäure-trans-4-[trans-4-(4-pentenyl)cyclohexyl]cyclohexylester;
3,4-Difluorbenzoesäure-trans-4-[trans-4-(4-pentenyl)cyclohexyl]cyclohexylester;
3-Chlor-4-fluorbenzoesäure-trans-4-[trans-4-(4-pentenyl)cyclohexyl]cyclohexylester;
4-Chlorbenzoesäure-trans-4-[trans-4-(4-pentenyl)cyclohexyl]cyclohexylester;
3-Fluor-4-chlorbenzoesäure-trans-4-[trans-4-(4-pentenyl)cyclohexyl]cyclohexylester;
trans-4-[trans-4-(4-Pentenyl)cyclohexyl]cyclohexyl-4-fluorbenzyläther;
trans-4-[trans-4-(4-Pentenyl)cyclohexyl]cyclohexyl-3,4-difluorbenzyläther;
trans-4-[trans-4-(4-Pentenyl)cyclohexyl]cyclohexyl-3-chlor-4-fluorbenzyläther;
trans-4-[trans-4-(4-Pentenyl)cyclohexyl]cyclohexyl-4-chlorbenzyläther;
trans-4-[trans-4-(4-Pentenyl)cyclohexyl]cyclohexyl-3-fluor-4-chlorbenzyläther;
1-[trans-4-[trans-4-(4Z-Hexenyl)cyclohexyl]cyclohexyl]-4-fluorbenzol;
1-[trans-4-[trans-4-(4Z-Hexenyl)cyclohexyl]cyclohexyl]-3,4-difluorbenzol;
1-[trans-4-[2-(trans-4-(4Z-Hexenyl)cyclohexyl)äthyl]cyclohexyl]-4-fluorbenzol;
1-[trans-4-[2-(trans-4-(4Z-Hexenyl)cyclohexyl)äthyl]cyclohexyl]-3,4-difluorbenzol;
1-[[trans-4-(trans-4-(4Z-Hexenyl)cyclohexyl)cyclohexyl]methoxy]-4-fluorbenzol;
1-[[trans-4-(trans-4-(4Z-Hexenyl)cyclohexyl)cyclohexyl]methoxy]-3,4-difluorbenzol;
1-[2-[trans-4-(trans-4-(4Z-Hexenyl)cyclohexyl)cyclohexyl]äthyl]-4-fluorbenzol;
1-[2-[trans-4-(trans-4-(4Z-Hexenyl)cyclohexyl)cyclohexyl]äthyl]-3,4-difluorbenzol;

4-Fluorbenzoesäure-trans-4-[trans-4-(4Z-hexenyl)cyclohexyl]cyclohexylester;
3,4-Difluorbenzoesäure-trans-4-[trans-4-(4Z-hexenyl)cyclohexyl]cyclohexylester;
trans-4-[trans-4-(4Z-Hexenyl)cyclohexyl]cyclohexyl-4-fluorbenzyläther;
trans-4-[trans-4-(4Z-Hexenyl)cyclohexyl]cyclohexyl-3,4-difluorbenzyläther.

Durch Weglassen der Stufen b) und c) können in analoger Weise auch die in Beispiel 3 genannten 3-Alkenylverbindungen und weitere 3-Alkenylderivate hergestellt werden.

Beispiel 10

Die nachstehend aufgeführten binären Mischungen wurden hergestellt und in einer TN-Zelle mit einem Plattenabstand von 8 µm bei 22 °C untersucht. Die entsprechenden Daten für 4-(trans-4-Pentylcyclohexyl)-benzonitril betragen: Klp. 54,6 °C, $V_{10}$ = 1,62V, $t_{on}$ = 30 ms, $t_{off}$ = 42 ms, Δ = 0,120.

Mischung 1

50 Gew.-% trans-4-(3-Butenyl)-1-(trans-4-äthoxycyclohexyl)cyclohexan,
50 Gew.-% 4-(trans-4-Pentylcyclohexyl)benzonitril;
Klp. 44,4 °C, $V_{10}$ = 1,66V, $t_{on}$ = 21 ms, $t_{off}$ = 31 ms, Δn = 0,082.

Mischung 2

50 Gew.-% trans-4-(3E-Pentenyl)-1-(trans-4-methoxycclohexyl)cyclohexan,
50 Gew.-% 4-(trans-4-Pentylcyclohexyl)benzonitril;
Smp. 44,6 °C, $V_{10}$ = 1,55V, $t_{on}$ = 22 ms, $t_{off}$ = 37 ms, Δn = 0,084.

Mischung 3

50 Gew.-% trans-4-(3E-Pentenyl)-1-(trans-4-äthoxycyclohexyl)cyclohexan.
50 Gew.-% 4-(trans-4-Pentylcyclohexyl)benzonitril;
Smp. 57,4 °C, $V_{10}$ = 2,11V, $t_{on}$ = 19 ms, $t_{off}$ = 28 ms, Δn = 0,094.

Vergleichsmischung 4

50 Gew.-% trans-4-Butylcyclohexancarbonsäure-trans-4-butylcyclohexylester.
50 Gew.-% 4-(trans-4-Pentylcyclohexyl)benzonitril:
Smp. 40,2 °C, $V_{10}$ = 1,39V, $t_{on}$ = 38 ms. $t_{off}$ = 63 ms. Δn = 0,08.

Vergleichsmischung 5

50 Gew.-% trans-4-Pentyl-1-(trans-4-methoxycyclohexyl)cyclohexan,
50 Gew.-% 4-(trans-4-Pentylcyclohexyl)benzonitril:
Smp. 39,7 °C, $V_{10}$ = 1,47V, $t_{on}$ = 35 ms, $t_{off}$ = 42 ms, Δn = 0,071.

Beispiel 11

Die nachstehend aufgeführten Mischungen wurden hergestellt und deren Eigenschaften gemessen. Die Messung der elektro-optischen Daten erfolgte sofern nicht anders angegeben, in einer TN-Zelle mit einem Plattenabstand von 6 µm bei 22 °C.

Mischung A

| | | |
|---|---|---|
| 10 | Gew.-% | 4-[trans-4-(1E-Propenyl)cyclohexyl]benzonitril, |
| 15 | " | 4-Aethoxy-1-[trans-4-(3E-Pentenyl)cyclohexyl]benzol, |
| 12 | " | trans-4-[trans-4-(3-Butenyl)cyclohexyl]cyclohexan-carbonitril, |
| 10 | " | trans-4-[trans-4-(3E-Pentenyl)cyclohexyl]cyclohexan-carbonitril, |
| 8 | " | trans-4-[trans-4-(4-Pentenyl)cyclohexyl]cyclohexan-carbonitril, |
| 8 | " | 1-[2-(trans-4-Butylcyclohexyl)äthyl]-4-(trans-4-pentylcyclohexyl)benzol, |
| 8 | " | trans-4-[trans-4-(3-Butenyl)cyclohexyl]cyclohexan-carbonsäure-4-fluorphenylester, |
| 8 | " | trans-4-[trans-4-(3E-Pentenyl)cyclohexyl]cyclohexan-carbonsäure-4-fluorphenylester, |
| 6 | " | trans-4-[trans-4-(4-Pentenyl)cyclohexyl]cyclohexan-carbonsäure-4-fluorphenylester, |
| 15 | " | trans-4-(3-Butenyl)-1-(trans-4-äthoxycyclohexyl)-cyclohexan; |

Smp. <-30 °C, Klp. 84 °C, nematisch; $V_{10}$ = 2,35V; $t_{on}$ (22 °C) = 15 ms, $t_{on}$ (-20 °C) = 309 ms, $t_{off}$ (22 °C) = 27 ms, $t_{off}$ (-20 °C) = 420 ms; Δm = 0,088.

Mischung B

10 Gew.-% 4-[trans-4-(1E-Propenyl)cyclohexyl]benzonitril,
8 " 4-(2E-Butenyloxy)-1-(trans-4-propylcyclohexyl)-benzol,
9 " trans-4-[trans-4-(3-Butenyl)cyclohexyl]cyclohexan-carbonitril,
6 " trans-4-[trans-4-(4-Pentenyl)cyclohexyl]cyclohexan-carbonitril,
12 " trans-4-Allyloxy-1-(trans-4-propylcyclohexyl)cyclo-hexan,
6 " r-1-Cyan-1-(3-butenyl)-cis-4-(trans-4-pentylcyclo-hexyl)cyclohexan,
3 " 2-Cyano-5-(trans-4-pentylcyclohexyl)pyrimidin,
3 " 4-(5-Butyl-2-pyrimidinyl)benzonitril,
6 " 4-[trans-5-(4-Pentenyl)-m-dioxan-2-yl]benzonitril,

7 " trans-4-Propylcyclohexancarbonsäure-4-[trans-4-(3E-pentenyl)cyclohexyl]phenylester,
10 " 1-[2-(trans-4-Butylcyclohexyl)äthyl]-4-(trans-4-pentylcyclohexyl)benzol,
4 " 4-[2-(trans-4-Butylcyclohexyl)äthyl]-4'-(trans-4-pentylcyclohexyl)biphenyl,
4 " 4-[2-(trans-4-Butylcyclohexyl)äthyl]-4'-[trans-4-(3E-pentenyl)cyclohexyl]biphenyl,
12 " trans-4-(3-Butenyl)-1-(trans-4-methoxycyclohexyl)-cyclohexan;

Smp. <-30 °C, Klp. 71 °C, nematisch; $V_{10}$ = 1,80V; $t_{on}$ = (22 °C) = 20 ms, $t_{on}$ (-20 °C) = 330 ms, $t_{off}$ - (22 °C) = 37 ms, $t_{off}$ (-20 °C) = 440 ms; $\Delta n$ = 0,090.

Mischung C

| | | |
|---|---|---|
| 8 | Gew.-% | 4-(2E-Butenyloxy)-1-(trans-4-propylcyclohexyl)-benzol, |
| 9 | " | trans-4-[trans-4-(3-Butenyl)cyclohexyl]cyclohexan-carbonitril, |
| 10 | " | trans-4-[trans-4-(3E-Pentenyl)cyclohexyl]cyclohexan-carbonitril, |
| 6 | " | trans-4-[trans-4-(4-Pentenyl)cyclohexyl]cyclohexan-carbonitril, |
| 12 | " | trans-4-Allyloxy-1-(trans-4-propylcyclohexyl)cyclo-hexan, |
| 6 | " | r-1-Cyan-1-(3-butenyl)-cis-4-(trans-4-pentylcyclo-hexyl)cyclohexan, |
| 3 | " | 2-Cyano-5-(trans-4-pentylcyclohexyl)pyrimidin, |
| 3 | " | 4-(5-Butyl-2-pyrimidinyl)benzonitril, |
| 6 | " | 4-[trans-5-(4-Pentenyl)-m-dioxan-2-yl]benzonitril, |
| 7 | " | trans-4-Propylcyclohexancarbonsäure-4-[trans-4-(3E-pentenyl)cyclohexyl]phenylester, |
| 10 | " | 1-[2-(trans-4-Butylcyclohexyl)äthyl]-4-(trans-4-pentylcyclohexyl)benzol, |
| 4 | " | 4-[2-(trans-4-Butylcyclohexyl)äthyl]-4'-(trans-4-pentylcyclohexyl)biphenyl, |
| 4 | " | 4-[2-(trans-4-Butylcyclohexyl)äthyl]-4'-[trans-4-(3E-pentenyl)cyclohexyl]biphenyl, |
| 12 | " | trans-4-(3-Butenyl)-1-(trans-4-methoxycyclohexyl)-cyclohexan; |

Smp. <-30 °C, Klp. 74 °C, nematisch; $V_{10}$ = 2,01V, $t_{on}$ (22 °C) = 34 ms, $t_{on}$ = (-20 °C) = 470 ms, $t_{off}$ - (22 °C) = 37 ms, $t_{off}$ (-20 °C) = 475 ms; $\Delta n$ = 0,083.

Mischung D

| | | |
|---|---|---|
| 10 | Gew.-% | 4-[trans-4-(1E-Propenyl)cyclohexyl]benzonitril, |
| 9 | " | trans-4-[trans-4-(3-Butenyl)cyclohexyl]cyclohexancarbonitril, |
| 6 | " | trans-4-[trans-4-(4-Pentenyl)cyclohexyl]cyclohexancarbonitril, |
| 12 | " | trans-4-Allyloxy-1-(trans-4-propylcyclohexyl)-cyclohexan, |
| 6 | " | r-1-Cyan-1-(3-butenyl)-cis-4-(trans-4-pentylcyclohexyl)cyclohexan, |
| 3 | " | 2-Cyano-5-(trans-4-pentylcyclohexyl)pyrimidin, |
| 3 | " | 4-(5-Butyl-2-pyrimidinyl)benzonitril, |
| 6 | " | 4-[trans-5-(4-Pentenyl)-m-dioxan-2-yl]benzonitril, |
| 8 | " | 4-Aethoxy-1-[2-(trans-4-pentylcyclohexyl)äthyl]-benzol, |
| 7 | " | trans-4-Propylcyclohexancarbonsäure-4-[trans-4-(3E-pentenyl)cyclohexyl]phenylester, |
| 10 | " | 1-[2-(trans-4-Butylcyclohexyl)äthyl]-4-(trans-4-pentylcyclohexyl)benzol, |
| 8 | " | 1-[trans-4-(trans-4-Propylcyclohexyl)cyclohexyl]-4-fluorbenzol, |
| 12 | " | trans-4-(3-Butenyl)-1-(trans-4-methoxycyclohexyl)-cyclohexan; |

Smp. <-30 °C, Klp. 62 °C, nematisch; $V_{10}$ = 1,79V, $t_{on}$ (22 °C) = 27 ms, $t_{on}$ = (-20 °C) = 480 ms, $t_{off}$ - (22 °C) = 50 ms, $t_{off}$ (-20 °C) = 480 ms; Δn = 0,081.

Mischung E

| | | |
|---|---|---|
| 10 | Gew.-% | 4-[trans-4-(1E-Propenyl)cyclohexyl]benzonitril, |
| 6 | " | 4-[trans-4-(3E-Propenyl)cyclohexyl]benzonitril, |
| 12 | " | 4-Aethoxy-1-[trans-4-(3E-pentenyl)cyclohexyl]benzol, |
| 5 | " | trans-4-[trans-4-(3-Butenyl)cyclohexyl]cyclohexancarbonitril, |
| 7 | " | trans-4-[trans-4-(3E-Pentenyl)cyclohexyl]cyclohexancarbonitril, |
| 12 | " | trans-4-Allyloxy-1-(trans-4-propylcyclohexyl)cyclohexan, |
| 6 | " | 4-[trans-4-(3E-Pentenyl)cyclohexyl]-4'-propylbiphenyl, |
| 6 | " | trans-4-Propylcyclohexancarbonsäure-4-[trans-4-(3E-pentenyl)cyclohexyl]phenylester, |
| 10 | " | 1-[2-(trans-4-Butylcyclohexyl)äthyl]-4-(trans-4-pentylcyclohexyl)benzol, |
| 4 | " | 4-[2-(trans-4-Butylcyclohexyl)äthyl]-4'-[trans-4-(3E-pentenyl)cyclohexyl]biphenyl, |
| 8 | " | trans-4-[trans-4-(3-Butenyl)cyclohexyl]cyclohexancarbonsäure-4-fluorphenylester, |
| 4 | " | trans-4-[trans-4-(3E-Pentenyl)cyclohexyl]cyclohexancarbonsäure-4-fluorphenylester, |
| 10 | " | trans-4-(3E-Pentenyl)-1-(trans-4-äthoxycyclohexyl)-cyclohexan; |

Smp. <-30 °C, Klp. 100 °C, nematisch; $V_{10}$ = 2,82V, $t_{on}$ (22 °C) = 12 ms, $t_{on}$ = (-20 °C) = 220 ms, $t_{off}$ - (22 °C) = 21 ms, $t_{off}$ (-20 °C) = 330 ms; $\Delta n$ = 0,105.

Mischung F

| | | |
|---|---|---|
| 9,40 | Gew.-% | 4-[trans-4-(1E-Propenyl)cyclohexyl]benzonitril, |
| 5,64 | " | 4-[trans-4-(3E-Pentenyl)cyclohexyl]benzonitril, |
| 11,28 | " | 4-Aethoxy-1-[trans-4-(3E-pentenyl)cyclohexyl]-benzol, |
| 4,70 | " | trans-4-[trans-4-(3-Butenyl)cyclohexyl]cyclohexancarbonitril, |
| 6,58 | " | trans-4-[trans-4-(3E-Pentenyl)cyclohexyl]cyclohexancarbonitril, |
| 11,28 | " | trans-4-Allyloxy-1-(trans-4-propylcyclohexyl) cyclohexan, |
| 6,00 | " | 2-Cyano-5-(trans-4-butylcyclohexyl)pyrimidin, |
| 5,64 | " | trans-4-Propylcyclohexancarbonsäure-4-[trans-4-(3E-pentenyl)cyclohexyl]phenylester, |
| 5,64 | " | 4-[trans-4-(3E-Pentenyl)cyclohexyl]-4'-propyl-biphenyl, |
| 9,40 | " | 1-[2-(trans-4-Butylcyclohexyl)äthyl]-4-(trans-4-pentylcyclohexyl)benzol, |
| 3,76 | " | 4-[2-(trans-4-Butylcyclohexyl)äthyl]-4'-[trans-4-(3E-pentenyl)cyclohexyl]biphenyl, |
| 7,52 | " | trans-4-[trans-4-(3-Butenyl)cyclohexyl]cyclohexancarbonsäure-4-fluorphenylester, |
| 3,76 | " | trans-4-[trans-4-(3E-Pentenyl)cyclohexyl]cyclohexancarbonsäure-4-fluorphenylester, |
| 9,40 | " | trans-4-(3E-Pentenyl)-1-(trans-4-äthoxycyclohexyl)cyclohexan; |

Smp. <-30 °C, Klp. 95 °C, nematisch; $V_{10}$ = 2,35V, $t_{on}$ (22 °C) = 16 ms, $t_{on}$ = (-20 °C) = 300 ms, $t_{off}$ - (22 °C) = 24 ms, $t_{off}$ (-20 °C) = 350 ms; Δn = 0,104.

Mischung G

| | | |
|---|---|---|
| 10 | Gew.-% | 4-[trans-4-(1E-Propenyl)cyclohexyl]benzonitril, |
| 12 | " | 4-(2E-Butenyloxy)-1-(trans-4-propylcyclohexyl)-benzol, |
| 5 | " | trans-4-[trans-4-(3-Butenyl)cyclohexyl]cyclohexan-carbonitril, |
| 7 | " | trans-4-[trans-4-(3E-Pentenyl)cyclohexyl]cyclohexan-carbonitril, |
| 6 | " | trans-4-[trans-4-(4-Pentenyl)cyclohexyl]cyclohexan-carbonitril, |
| 12 | " | trans-4-Allyloxy-1-(trans-4-propylcyclohexyl)cyclo-hexan, |
| 8 | " | trans-4-Propylcyclohexancarbonsäure-4-[trans-4-(3E-pentenyl)cyclohexyl]phenylester, |
| 10 | " | 1-[2-(trans-4-Butylcyclohexyl)äthyl]-4-(trans-4-pentylcyclohexyl)benzol, |
| 5 | " | 4-[2-(trans-4-Butylcyclohexyl)äthyl]-4-(trans-4-pentylcyclohexyl)biphenyl, |
| 3 | " | 5-(trans-4-Pentylcyclohexyl)-2-[4-(trans-4-propyl-cyclohexyl)phenyl]pyrimidin, |
| 4 | " | trans-4-[trans-4-(3-Butenyl)cyclohexyl]cyclohexan-carbonsäure-4-fluorphenylester, |
| 8 | " | trans-4-[trans-4-(3E-Pentenyl)cyclohexyl]cyclohexan-carbonsäure-4-fluorphenylester, |
| 10 | " | trans-4-(3E-Pentenyl)-1-(trans-4-äthoxycyclohexyl)-cyclohexan; |

Smp. <-30 °C, Klp. 109 °C, nematisch: $V_{10}$ = 2,84V, $t_{on}$ (22 °C) = 16 ms, $t_{on}$ = (-20 °C) = 336 ms, $t_{off}$ - (22 °C) = 26 ms, $t_{off}$ (-20 °C) = 370 ms; $\Delta n$ = 0,096.

Mischung H

| | | |
|---|---|---|
| 15 | Gew.-% | trans-4-[trans-4-(3-Butenyl)cyclohexyl]cyclohexancarbonitril, |
| 12 | " | trans-4-[trans-4-(3E-Pentenyl)cyclohexyl]cyclohexancarbonitril, |
| 10 | " | trans-4-[trans-4-(4-Pentenyl)cyclohexyl]cyclohexancarbonitril, |
| 10 | " | 4-[trans-4-(1E-Propenyl)cyclohexyl]benzonitril, |
| 20 | " | trans-4-Allyloxy-1-(trans-4-propylcyclohexyl)-cyclohexan, |
| 10 | " | trans-4-[trans-4-(3-Butenyl)cyclohexyl]cyclohexancarbonsäure-4-fluorphenylester, |
| 8 | | trans-4-[trans-4-(4-Pentenyl)cyclohexyl]cyclohexancarbonsäure-4-fluorphenylester; |
| 15 | " | 4-Aethoxy-1-[trans-4-(3E-pentenyl)cyclohexyl]-benzol, |

Smp. <-30 °C, Klp. (N-I) 71 °C; $V_{10}$ = 2,10V, $t_{on}$ (22 °C) = 19 ms, $t_{on}$ (-20 °C) = 477 ms, $t_{off}$ (22 °C) = 31 ms, $t_{off}$ (-20 °C) = 391 ms, $\Delta n$ = 0,084.

Beispiel 12

Die diamagnetische Anisotropie der erfindungsgemässen Verbindungen ist annähernd null und der Absolutwert der dielektrischen Anisotropie ist klein. Zur Untersuchung der Eigenschaften wurden daher zwecks Erhöhung der diamagnetischen Anisotropie binäre Mischungen (BM) mit niederviskosen, unpolaren Phenylcyclohexanen hergestellt. Die Messung der physikalischen Eigenschaften erfolgte bei einer Temperatur 10 °C unter dem jeweiligen Klärpunkt, sofern nicht anders angegeben. Werte mit einem Stern * sind extrapolierte Werte. Die entsprechenden Daten der verwendeten Phenylcyclohexane sind:
für 4-(2E-Butenyl)oxy-1-(trans-4-propylcyclohexyl)- benzol:
Smp. (C-N) 42,4 °C, Klp. (N-I) 57,5 °C, $k_{11}$ = 10,9 pN, $k_{22}$ = 5,60 pN, $k_{33}$ = 12,4 pN, $\Delta\epsilon$ = -0,33, $\Delta n$ = 0,090, $\eta$ = 5,3 cp, $\eta$ (22 °C) = 13,5 cp*, $\gamma_1$ = 22 cp, $\gamma_1$ (22 °C) = 86 cp*;
für 4-Aethoxy-1-[trans-4-(3E-pentenyl)cyclohexyl]benzol:
Smp. (C-N) 49,4 °C, Klp. (N-I) 61,8 °C, $k_{11}$ = 10,1 pN, $k_{22}$ = 4,82 pN, $k_{33}$ = 15,7 pN, $\Delta\epsilon$ = -0,27, $\Delta n$ = 0,095, $\eta$ = 4,7 cp, $\gamma_1$ = 25 cp.

BM-1

50 Mol-% trans-4-(3-Butenyl)-1-(trans-4-äthoxycyclohexyl)cyclohexan,
50 Mol-% 4-(2E-Butenyl)oxy-1-(trans-4-propylcyclohexyl)benzol;
Smp. <15 °C, Klp. (N-I) 44,5 °C, $k_{11}$ = 7,07 pN, $k_{22}$ = 3,78 pN, $k_{33}$ = 8,08 pN, $\Delta\epsilon$ = -0,31, $\Delta n$ = 0,065, $\eta$ = 6,8 cp, $\eta$ (22 °C) = 10,3 cp, $\gamma_1$ = 41 cp, $\gamma_1$ (22 °C) = 62 cp.

BM-2

50 Mol-% trans-4-(3E-Pentenyl)-1-(trans-4-äthoxycyclohexl)cyclohexan,
50 Mol-% 4-(2E-Butenyl)oxy-1-(trans-4-propylcyclohexyl)benzol;
Smp. <15 °C, Klp. (N-I) 58,6 °C, $k_{11}$ = 8,71 pN, $k_{22}$ = 4,13 pN, $k_{33}$ = 11,2 pN, $\Delta\epsilon$ = -0,21, $\Delta n$ = 0,070, $\eta$ = 5,2 cp, $\eta$ (22 °C) = 11,4 cp, $\gamma_1$ = 20 cp, $\gamma_1$ (22 °C) = 66 cp.

BM-3

50 Mol-% trans-4-(3E-Pentenyl)-1-(trans-4-methoxycyclohexyl)cyclohexan,
50 Mol-% 4-(2E-Butenyl)oxy-1-(trans-4-propylcyclohexyl)benzol;
Smp. <15 °C, Klp. (N-I) 45,0 °C, $k_{11}$ = 6,73 pN, $k_{22}$ = 3,69 pN, $k_{33}$ = 7,94 pN, $\Delta\epsilon$ = -0,20, $\Delta n$ = 0,066, $\eta$ = 7,8 cp, $\eta$ (22 °C) = 11,7 cp, $\gamma_1$ = 27 cp, $\gamma_1$ (22 °C) = 53 cp.

BM-4

50 Mol-% trans-4-(3-Butenyl)-1-(trans-4-äthoxycyclohexyl)cyclohexan,
50 Mol-% 4-Aethoxy-1-[trans-4-(3E-pentenyl)cyclohexyl]benzol;
Smp. <15 °C, Klp. (N-I) 48,2 °C, $k_{11}$ = 7,16 pN, $k_{22}$ = 4,05 pN, $k_{33}$ = 9,88 pN, $\Delta\epsilon$ = -0,31, $\Delta n$ = 0,068, $\eta$ = 6,3 cp, $\eta$ (22 °C) = 9,8 cp, $\gamma_1$ = 22 cp, $\gamma_1$ (22 °C) = 47 cp.

**Patentansprüche**

1. Verbindungen der allgemeinen Formel

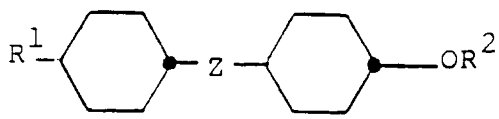

I

worin Z eine einfache Kovalenzbindung oder $-CH_2CH_2$ -darstellt, $R^1$ 1E-Alkenyl mit 2-10 Kohlenstoffatomen oder 3E-Alkenyl mit 4-10 Kohlenstoffatomen bezeichnet und $R^2$ Alkyl mit 1-10 Kohlenstoffatomen, 2E-Alkenyl mit 3-10 Kohlenstoffatomen oder 3-Alkenyl mit 4-10 Kohlenstoffatomen bedeutet.

2. Verbindungen nach Anspruch 1, dadurch gekennzeichnet, dass $R^1$ 1E-Alkenyl mit 2-7 Kohlenstoffatomen oder 3E-Alkenyl mit 4-7 Kohlenstoffatomen bezeichnet.

3. Verbindungen nach Anspruch 1 oder 2, dadurch gekennzeichnet, dass $R^2$ Alkyl mit 1-6 Kohlenstoffatomen, 2E-Alkenyl mit 3-6 Kohlenstoffatomen oder 3-Alkenyl mit 4-6 Kohlenstoffatomen bedeutet.

4. Verbindungen nach einem der Ansprüche 1 bis 3, dadurch gekennzeichnet, dass $R^1$ und $R^2$ geradkettige Reste bedeuten.

5. Flüssigkristallines Gemisch mit mindestens 2 Komponenten, dadurch gekennzeichnet, dass mindestens eine Komponente eine Verbindung der in Anspruch 1 definierten Formel I ist und dass der Anteil an Verbindungen der Formel I im gesamtgemisch 1-70 Gew.-%, vorzugsweise 3-40 Gew.-% beträgt.

6. Flüssigkristallines Gemisch nach Anspruch 6, dadurch gekennzeichnet, dass es eine oder mehrere Verbindungen der Formel I und eine oder mehrere Verbindungen mit positiver dielektrischer Anisotropie enthält.

7. Flüssigkristallines Gemisch nach Anspruch 5 oder 6, dadurch gekennzeichnet, dass es eine oder mehrere Verbindungen der Formel I und eine oder mehrere Verbindungen aus der Gruppe der Verbindungen der allgemeinen Formeln

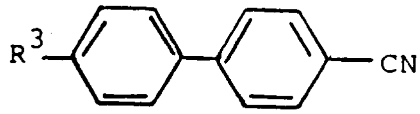

II

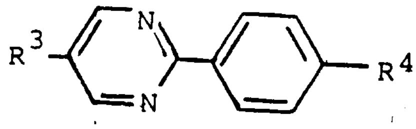

III

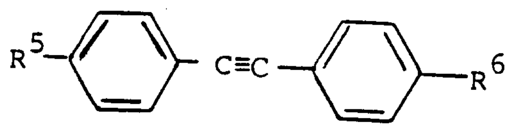

IV

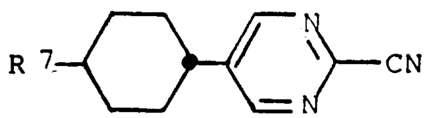

V

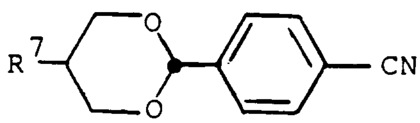

VI

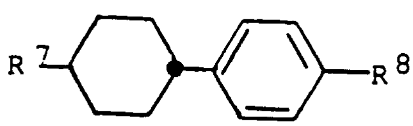

VII

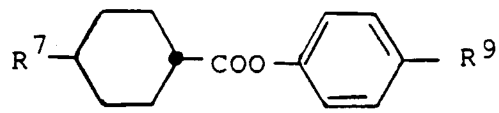

VIII

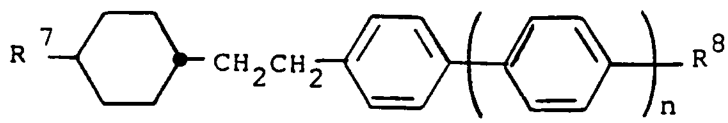

IX

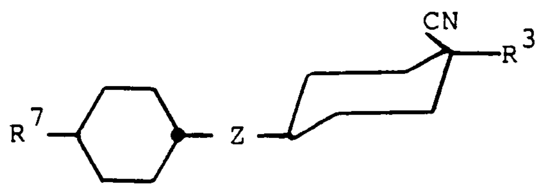

X

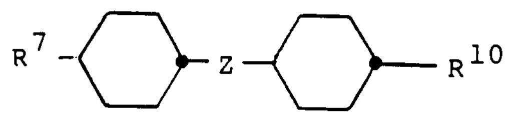

XI

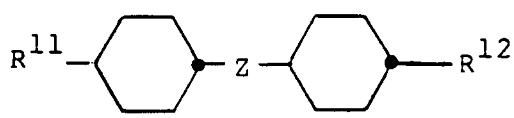

XII

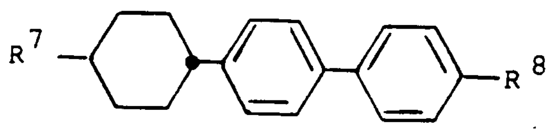

XIII

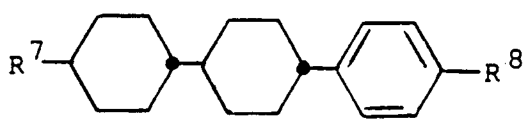

XIV

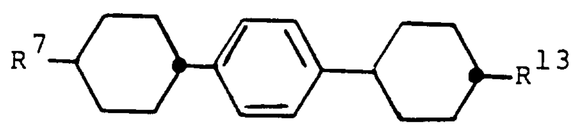

XV

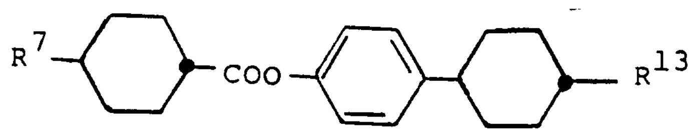

XVI

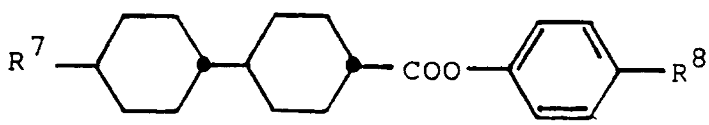

XVII

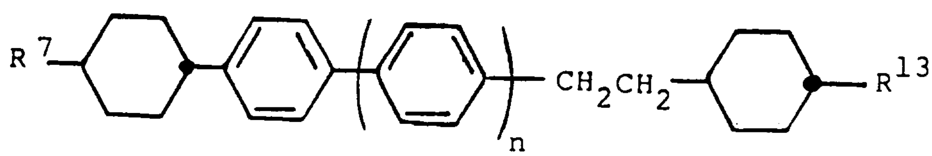

XVIII

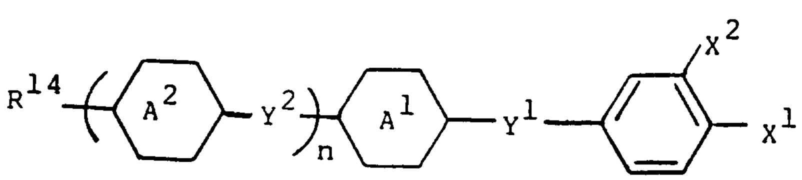

XIX

worin $R^3$ Alkyl, 3E-Alkenyl oder 4-Alkenyl bedeutet; $R^4$ Cyano oder Fluor darstellt; $R^5$ und $R^6$ Alkyl oder Alkoxy bezeichnen; $R^7$ und $R^{13}$ unabhängig voneinander Alkyl, 1E-Alkenyl, 3E-Alkkenyl oder 4-Alkenyl bedeuten; $R^8$ Cyano, Alkyl, 1E-Alkenyl, 3E-Alkenyl, 4-Alkenyl, Alkoxy, 2E-Alkenyloxy oder 3-Alkenyloxy bezeichnet; $R^9$ Alkoxy, 2E-Alkenyloxy oder 3-Alkenyloxy bedeutet; n für die Zahl 0 oder 1 steht; Z eine einfache Kovalenzbindung oder $-CH_2CH_2-$ darstellt; $R^{10}$ Cyano, Alkyl, 1E-Alkenyl, 3E-Alkenyl oder 4-Alkenyl bedeutet; $R^{11}$ Alkyl oder 4-Alkenyl bezeichnet; $R^{12}$ Alkoxy, 2E-Alkenyloxy oder 3-Alkenyloxy darstellt; $X^1$ Fluor oder Chlor und $X^2$ Wasserstoff, Fluor oder Chlor bezeichnen; $R^{14}$ Alkyl, 3E-Alkenyl, 4-Alkenyl, Alkoxy, 2E-Alkenyloxy oder 3-Alkenyloxy bedeutet; eine der Gruppen $Y^1$ und $Y^2$ eine einfache Kovalenzbindung, -COO-, -OOC-, $-CH_2CH_2-$, $-CH_2O-$ oder $-OCH_2-$ und die andere der Gruppen $Y^1$ und $Y^2$ eine einfache Kovalenzbindung bedeutet; und die Ringe $A^1$ und $A^2$ unabhängig voneinander substituiertes oder unsubstituiertes trans-1,4-Cyclohexylen, in welchem gegebenenfalls 2 nicht benachbarte $CH_2$-Gruppen durch Sauerstoff ersetzt sind, oder substituiertes oder unsubstituiertes 1,4-Phenylen, in welchem gegebenenfalls 1 oder 2 CH -Gruppen durch Stickstoff ersetzt sind, darstellen,
enthält.

**8.** Flüssigkristallzelle aus einer zwischen zwei mit Elektroden und Oberflächenorientierungen versehenen Platten und Polarisatoren angeordneten verdrillten nematischen Flüssigkristallschicht mit positiver dielektrischer Anisotropie, dadurch gekennzeichnet, dass der Flüssigkristall eine oder mehrere Verbindungen der in Anspruch 1 definierten Formel I enthält.

**9.** Verwendung der Verbindungen der in Anspruch 1 definierten Formel I für elektro-optische Zwecke.

**Claims**

**1.** Compounds of the general formula

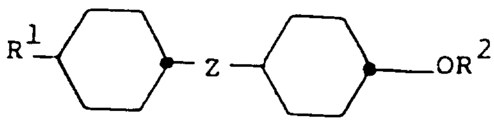

I

wherein Z represents a single covalent bond or $-CH_2CH_2-$, $R^1$ denotes 1E-alkenyl with 2-10 carbon atoms or 3E-alkenyl with 4-10 carbon atoms and $R^2$ signifies alkyl with 1-10 carbon atoms, 2E-alkenyl with 3-10 carbon atoms or 3-alkenyl with 4-10 carbon atoms.

**2.** Compounds according to claim 1, characterized in that $R^1$ denotes 1E-alkenyl with 2-7 carbon atoms or 3E-alkenyl with 4-7 carbon atoms.

**3.** Compounds according to claim 1 or 2, characterized in that $R^2$ signifies alkyl with 1-6 carbon atoms, 2E-alkenyl with 3-6 carbon atoms or 3-alkenyl with 4-6 carbon atoms.

**4.** Compounds according to any one of claims 1 to 3, characterized in that $R^1$ and $R^2$ signify straight-chain residues.

**5.** A liquid crystalline mixture with at least 2 components, characterized in that at least one component is a compound of formula I defined in claim 1 and the amount of compounds of formula I in the total mixture amounts to 1-70 wt.%, preferably 3-40 wt.%.

**6.** A liquid crystalline mixture according to claim 6, characterized in that it contains one or more compounds of formula I and one or more compounds with positive dielectric anisotropy.

**7.** A liquid crystalline mixture according to claim 5 or 6, characterized in that it contains one or more compounds of formula I and one or more compounds from the group of compounds of the general formulae

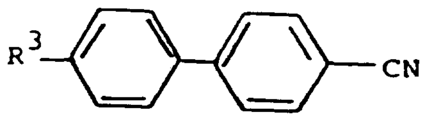

II

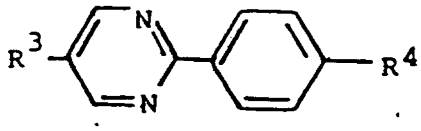

III

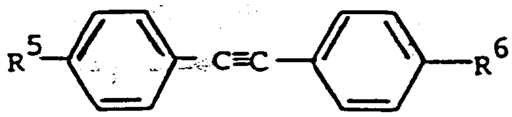

IV

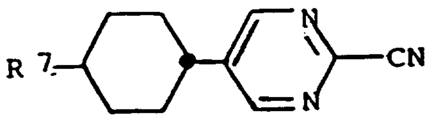

V

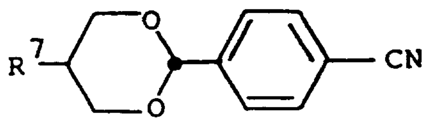

VI

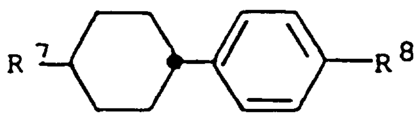

VII

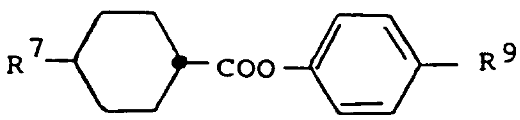

VIII

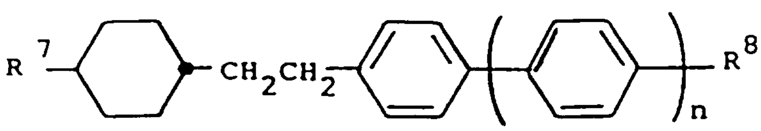

IX

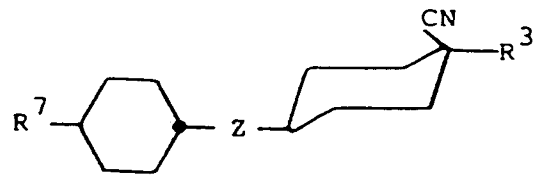

X

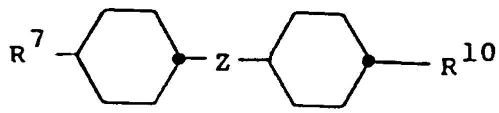

XI

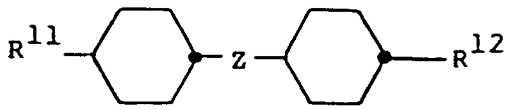

XII

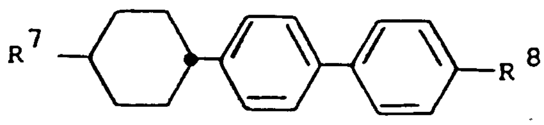

XIII

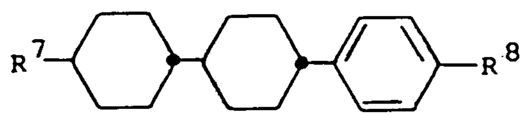

XIV

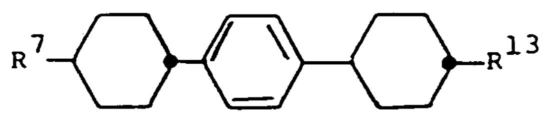

XV

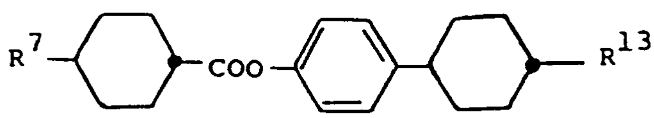

XVI

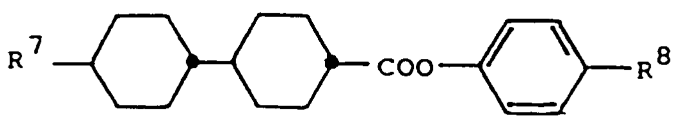

XVII

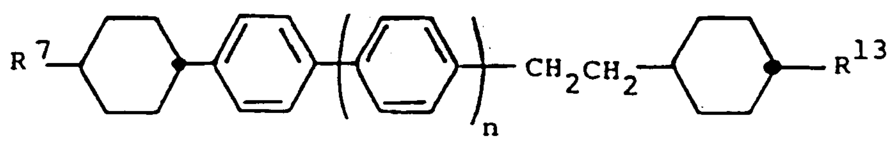

XVIII

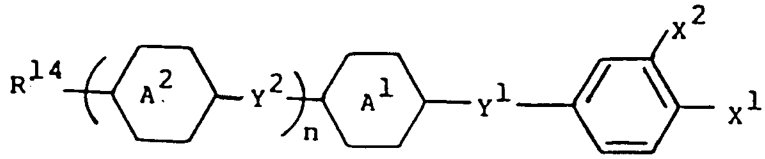

XIX

wherein $R^3$ signifies alkyl, 3E-alkenyl or 4-alkenyl; $R^4$ represents cyano or fluoro; $R^5$ and $R^6$ denote alkyl or alkoxy; $R^7$ and $R^{13}$ each independently signify alkyl, 1E-alkenyl, 3E-alkenyl or 4-alkenyl; $R^8$ denotes cyano, alkyl, 1E-alkenyl, 3E-alkenyl, 4-alkenyl, alkoxy, 2E-alkenyloxy or 3-alkenyloxy; $R^9$ signifies alkoxy, 2E-alkenyloxy or 3-alkenyloxy: n stands for the number 0 or 1; Z represents a single covalent bond or $-CH_2CH_2-$; $R^{10}$ signifies cyano alkyl, 1E-alkenyl, 3E-alkenyl or 4-alkenyl; $R^{11}$ denotes

alkyl or 4-alkenyl; $R^{12}$ represents alkoxy, 2E-alkenyloxy or 3-alkenyloxy; $X^1$ denotes fluorine or chlorine and $X^2$ denotes hydrogen, fluorine or chlorine: $R^{14}$ signifies alkyl, 3E-alkenyl, 4-alkenyl, alkoxy, 2E-alkenyloxy or 3-alkenyloxy; one of the groups $Y^1$ and $Y^2$ signifies a single covalent bond, -COO-, -OOC-, $-CH_2CH_2-$, $-CH_2O-$ or $-OCH_2-$ and the other of the groups $Y^1$ and $Y^2$ signifies a single covalent bond; and rings $A^1$ and $A^2$ each independently represent substituted or unsubstituted trans-1,4--cyclohexylene in which optionally 2 non-adjacent $CH_2$ groups are replaced by oxygen or substituted or unsubstituted 1,4-phenylene in which optionally 1 $CH_2$ group or 2 $CH_2$ groups is/are replaced by nitrogen.

8. A liquid crystal cell from a twisted nematic liquid crystal layer with positive dielectric anisotropy arranged between two plates provided with electrodes and surface orientations and polarizers, characterized in that the liquid crystal contains one or more compounds of formula I defined in claim 1.

9. The use of compounds of formula I defined in claim 1 for electro-optical purposes.

## Revendications

1. Composés de formule générale

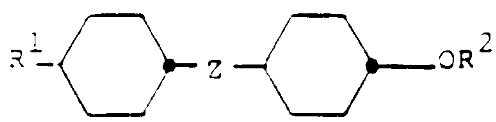

I

dans laquelle Z représente une simple liaison covalente ou $-CH_2CH_2-$, $R^1$ représente un groupe 1E-alcényle ayant 2-10 atomes de carbone ou 3E-alcényle ayant 4-10 atomes de carbone, et $R^2$ représente un groupe alkyle ayant 1-10 atomes de carbone, 2E-alcényle ayant 3-10 atomes de carbone, ou 3-alcényle ayant 4-10 atomes de carbone.

2. Composés selon la revendication 1, caractérisés en ce que $R^1$ représente un groupe 1E-alcényle ayant 2-7 atomes de carbone ou 3E-alcényle ayant 4-7 atomes de carbone.

3. Composés selon la revendication 1 ou 2, caractérisés en ce que $R^2$ représente un groupe alkyle ayant 1-6 atomes de carbone, 2E-alcényle ayant 3-6 atomes de carbone ou 3-alcényle ayant 4-6 atomes de carbone.

4. Composés selon l'une des revendications 1 à 3, caractérisés en ce que $R^1$ et $R^2$ représentent des radicaux à chaîne droite.

5. Composition de cristaux liquides à au moins 2 composants, caractérisée en ce qu'au moins un composant est un composé de formule I définie dans la revendication 1, et en ce que la proportion des composés de formule I dans la composition totale va de 1 à 70 % en poids, de préférence de 3 à 40 % en poids.

6. Composition de cristaux liquides selon la revendication 6, caractérisée en ce qu'elle contient un ou plusieurs composés de formule I et un ou plusieurs composés à anisotropie diélectrique positive

7. Composition de cristaux liquides selon la revendication 5 ou 6, caractérisée en ce qu'elle contient un ou plusieurs composés de formule I et un ou plusieurs composés choisis parmi les composés de formules générales

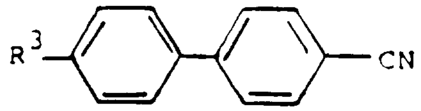

II

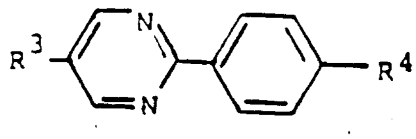

III

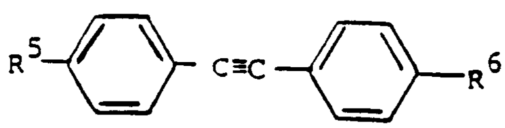

IV

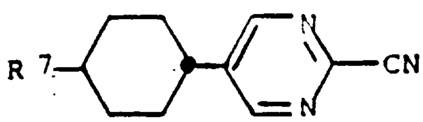

V

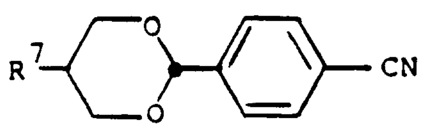

VI

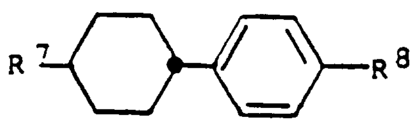

VII

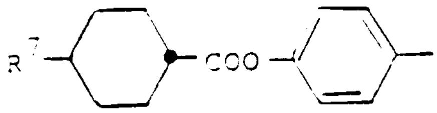

VIII

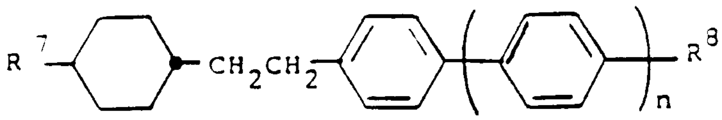

IX

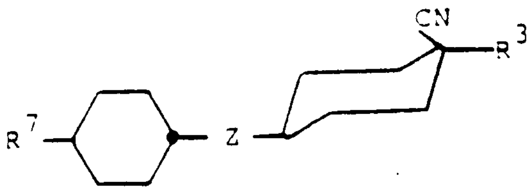

X

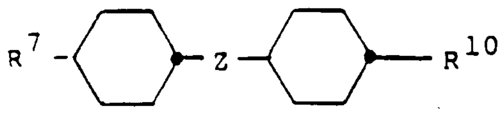

XI

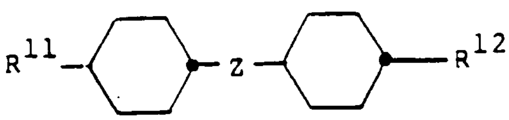

XII

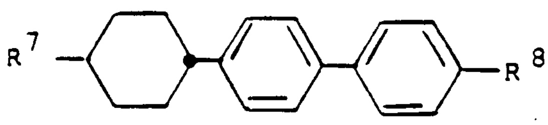

XIII

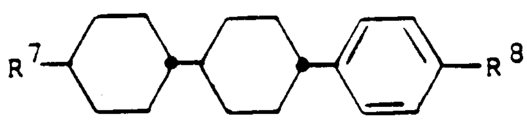

XIV

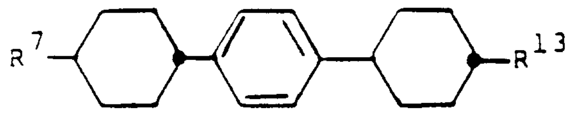

XV

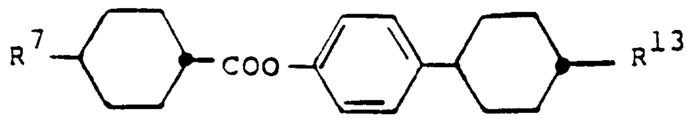

XVI

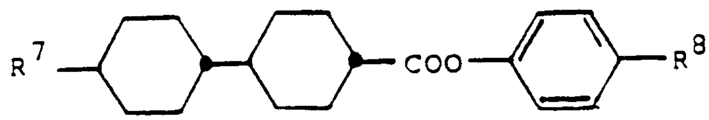

XVII

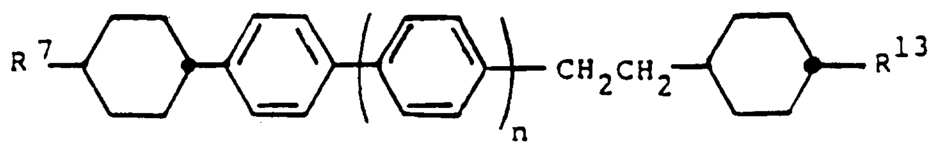

XVIII

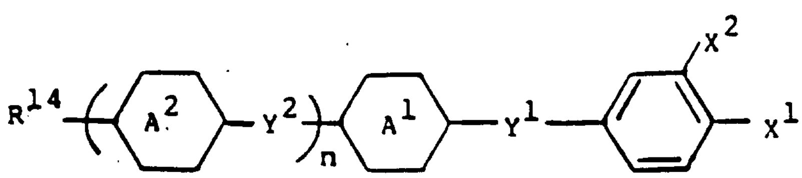

XIX

dans lesquelles $R^3$ représente un groupe alkyle, 3E-alcényle ou 4-alcényle; $R^4$ représente le groupe cyano ou un atome de fluor; $R^5$ et $R^6$ représentent un groupe alkyle ou alcoxy; $R^7$ et $R^{13}$ représentent, indépendamment l'un de l'autre, un groupe alkyle, 1E-alcényle, 3E-alcényle ou 4-alcényle; $R^8$ représente un groupe cyano, alkyle, 1E-alcényle, 3E-alcényle, 4-alcényle, alcoxy, 2E-alcényloxy ou 3-alcényloxy; $R^9$ représente un groupe alcoxy, 2E-alcényloxy ou 3-alcényloxy; n représente le nombre 0 ou 1; Z représente une simple liaison covalente; $R^{10}$ représente un groupe cyano, alkyle, 1E-alcényle, 3E-alcényle ou 4-alcényle; $R^{11}$ représente un groupe alkyle ou 4-alcényle; $R^{12}$ représente un groupe alcoxy, 2E-alcényloxy ou 3-alcényloxy; $X^1$ représente un atome de fluor ou de chlore; et $X^2$ représente un atome d'hydrogène, de fluor ou de chlore; $R^{14}$ représente un groupe alkyle, 3E-alcényle, 4-alcényle, alcoxy, 2E-alcényloxy ou 3-alcényloxy; l'un des groupes $Y^1$ et $Y^2$ représente une simple liaison covalente, -COO-, -OOC-, $-CH_2CH_2-$, $-CH_2O-$ ou $-OCH_2-$ et l'autre des groupes $Y^1$ et $Y^2$ représente une simple liaison covalente; et les cycles $A^1$ et $A^2$ représentent, indépendamment l'un de l'autre, un radical trans-1,4-cyclohexylène substitué ou non substitué, dans lequel éventuellement 2 groupes $CH_2$ non adjacents sont remplacés par des atomes d'oxygène, ou un radical 1,4-phénylène substitué ou non substitué, dans lequel éventuellement 1 ou 2 groupes CH sont remplacés par un atome d'azote.

**8.** Cellule à cristaux liquides constituée d'une couche de cristaux liquides nématiques en hélice, disposée entre deux polariseurs et deux plaques munies d'électrodes et d'orientations superficielles, caractérisée en ce que le cristal liquide contient un ou plusieurs composés de formule I définie dans la revendication 1.

**9.** Utilisation des composés de formule I définie dans la revendication 1, à des fins électro-optiques.